(19) **Europäisches Patentamt / European Patent Office / Office européen des brevets**

(11) **EP 3 635 128 B1**

(12) # EUROPEAN PATENT SPECIFICATION

(45) Date of publication and mention of the grant of the patent:
**25.08.2021 Bulletin 2021/34**

(21) Application number: **18726173.0**

(22) Date of filing: **30.05.2018**

(51) Int Cl.:
*C12Q 1/37* *(2006.01)*

(86) International application number:
**PCT/EP2018/064126**

(87) International publication number:
**WO 2018/219978 (06.12.2018 Gazette 2018/49)**

(54) **CONTINUOUS SPECTROMETRIC ASSAYS FOR MEASURING MEPRIN ACTIVITY USING AN AUXILIARY ENZYME**

KONTINUIERLICHES SPEKTROMETRISCHES ASSAY ZUR MESSUNG DER MEPRINAKTIVITÄT UNTER VERWENDUNG EINES HILFSENZYMS

ANALYSE SPECTROMÉTRIQUE CONTINUE POUR MESURER L'ACTIVITÉ DE LA MÉPRINE AU MOYEN D'UNE ENZYME AUXILIAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2017 EP 17173351**

(43) Date of publication of application:
**15.04.2020 Bulletin 2020/16**

(73) Proprietor: **Vivoryon Therapeutics AG**
**06120 Halle (Saale) (DE)**

(72) Inventors:
  • **SCHLENZIG, Dagmar**
    **06120 Halle (Saale) (DE)**
  • **HEMPEL, Marvin**
    **06108 Halle (Saale) (DE)**
  • **DEMUTH, Hans-Ulrich**
    **06120 Halle (Saale) (DE)**
  • **SCHULZE, Anja**
    **06114 Halle (Saale) (DE)**
  • **SCHILLING, Stephan**
    **06130 Halle (Saale) (DE)**
  • **RAMSBECK, Daniel**
    **06114 Halle (Saale) (DE)**
  • **WERMANN, Michael**
    **06118 Halle (Saale) (DE)**

(74) Representative: **Maikowski & Ninnemann Patentanwälte Partnerschaft mbB**
**Postfach 15 09 20**
**10671 Berlin (DE)**

(56) References cited:
**WO-A1-2007/003594   WO-A1-2011/076854**

• **FRANCK MADOUX ET AL: "Development of high throughput screening assays and pilot screen for inhibitors of metalloproteases meprin [alpha] and [beta]", BIOPOLYMERS, vol. 102, no. 5, 20 September 2014 (2014-09-20), pages 396-406, XP055307277, US ISSN: 0006-3525, DOI: 10.1002/bip.22527**
• **CHRISTOPH BECKER-PAULY ET AL: "Mapping orphan proteases by proteomics: Meprin metalloproteases deciphered as potential therapeutic targets", PROTEOMICS - CLINICAL APPLICATIONS, vol. 8, no. 5-6, 1 June 2014 (2014-06-01), pages 382-388, XP055414741, DE ISSN: 1862-8346, DOI: 10.1002/prca.201300079**
• **SCHILLING S ET AL: "Continuous Spectrometric Assays for Glutaminyl Cyclase Activity", ANALYTICAL BIOCHEMISTRY, ELSEVIER, AMSTERDAM, NL, vol. 303, no. 1, 1 April 2002 (2002-04-01) , pages 49-56, XP027227070, ISSN: 0003-2697 [retrieved on 2002-04-01]**
• **RUSSELL L. WOLZ ET AL: "Mapping the active site of meprin-A with peptide substrates and inhibitors", BIOCHEMISTRY, vol. 30, no. 34, 27 August 1991 (1991-08-27), pages 8488-8493, XP055414746, US ISSN: 0006-2960, DOI: 10.1021/bi00098a029**

- KRUSE M-N ET AL: "Human meprin [alpha] and [beta] homo-oligomers: Cleavage of basement membrane proteins and sensitivity to metalloprotease inhibitors", BIOCHEMICAL JOURNAL, PORTLAND PRESS LTD, GB, vol. 378, no. 2, 1 March 2004 (2004-03-01) , pages 383-389, XP002364593, ISSN: 0264-6021, DOI: 10.1042/BJ20031163
- J. BIEN ET AL: "The Metalloprotease Meprin Generates Amino Terminal-truncated Amyloid Peptide Species", JOURNAL OF BIOLOGICAL CHEMISTRY, vol. 287, no. 40, 28 September 2012 (2012-09-28), pages 33304-33313, XP055202580, ISSN: 0021-9258, DOI: 10.1074/jbc.M112.395608 cited in the application
- T. JEFFERSON ET AL: "Metalloprotease Meprin Generates Nontoxic N-terminal Amyloid Precursor Protein Fragments in Vivo", JOURNAL OF BIOLOGICAL CHEMISTRY, vol. 286, no. 31, 6 June 2011 (2011-06-06) , pages 27741-27750, XP055138563, ISSN: 0021-9258, DOI: 10.1074/jbc.M111.252718
- CLAUDIA BRODER ET AL: "The metalloproteases meprin [alpha] and meprin [beta]: unique enzymes in inflammation, neurodegeneration, cancer and fibrosis", BIOCHEMICAL JOURNAL, vol. 12, no. 2, 1 March 2013 (2013-03-01), pages 390-264, XP055307276, GB ISSN: 0264-6021, DOI: 10.1042/BJ20121751 cited in the application
- XU ET AL: "Novel Inhibitor for Prolyl Tripeptidyl Aminopeptidase from Porphyromonas gingivalis and Details of Substrate-recognition Mechanism", JOURNAL OF MOLECULAR BIOLOGY, ACADEMIC PRESS, UNITED KINGDOM, vol. 375, no. 3, 22 December 2007 (2007-12-22), pages 708-719, XP022400112, ISSN: 0022-2836, DOI: 10.1016/J.JMB.2007.09.077
- TAMARA JEFFERSON ET AL: "The substrate degradome of meprin metalloproteases reveals an unexpected proteolytic link between meprin [beta] and ADA", CMLS CELLULAR AND MOLECULAR LIFE SCIENCES, BIRKHÄUSER-VERLAG, BA, vol. 70, no. 2, 1 September 2012 (2012-09-01), pages 309-333, XP035157522, ISSN: 1420-9071, DOI: 10.1007/S00018-012-1106-2

SEQUENCE LISTING

<110> Fraunhofer-Gesellschaft zur Förderung der angewandten Forschung e. V.

<120> Continuous spectrometric assays for measuring meprin activity using an auxiliary enzyme

<130> FHG224WO

<160> 16

<170> PatentIn version 3.5

<210> 1
<211> 746
<212> PRT
<213> Human

<400> 1

```
Met Ala Trp Ile Arg Ser Thr Cys Ile Leu Phe Phe Thr Leu Leu Phe
1               5                   10                  15

Ala His Ile Ala Ala Val Pro Ile Lys Tyr Leu Pro Glu Glu Asn Val
            20                  25                  30

His Asp Ala Asp Phe Gly Glu Gln Lys Asp Ile Ser Glu Ile Asn Leu
            35                  40                  45

Ala Ala Gly Leu Asp Leu Phe Gln Gly Asp Ile Leu Leu Gln Lys Ser
    50                  55                  60

Arg Asn Gly Leu Arg Asp Pro Asn Thr Arg Trp Thr Phe Pro Ile Pro
65                  70                  75                  80

Tyr Ile Leu Ala Asp Asn Leu Gly Leu Asn Ala Lys Gly Ala Ile Leu
                85                  90                  95

Tyr Ala Phe Glu Met Phe Arg Leu Lys Ser Cys Val Asp Phe Lys Pro
            100                 105                 110

Tyr Glu Gly Glu Ser Ser Tyr Ile Ile Phe Gln Gln Phe Asp Gly Cys
            115                 120                 125

Trp Ser Glu Val Gly Asp Gln His Val Gly Gln Asn Ile Ser Ile Gly
    130                 135                 140

Gln Gly Cys Ala Tyr Lys Ala Ile Ile Glu His Glu Ile Leu His Ala
145                 150                 155                 160

Leu Gly Phe Tyr His Glu Gln Ser Arg Thr Asp Arg Asp Asp Tyr Val
                165                 170                 175
```

```
Asn Ile Trp Trp Asp Gln Ile Leu Ser Gly Tyr Gln His Asn Phe Asp
            180                 185                 190

Thr Tyr Asp Asp Ser Leu Ile Thr Asp Leu Asn Thr Pro Tyr Asp Tyr
            195                 200                 205

Glu Ser Leu Met His Tyr Gln Pro Phe Ser Phe Asn Lys Asn Ala Ser
    210                 215                 220

Val Pro Thr Ile Thr Ala Lys Ile Pro Glu Phe Asn Ser Ile Ile Gly
225                 230                 235                 240

Gln Arg Leu Asp Phe Ser Ala Ile Asp Leu Glu Arg Leu Asn Arg Met
                245                 250                 255

Tyr Asn Cys Thr Thr Thr His Thr Leu Leu Asp His Cys Thr Phe Glu
            260                 265                 270

Lys Ala Asn Ile Cys Gly Met Ile Gln Gly Thr Arg Asp Asp Thr Asp
            275                 280                 285

Trp Ala His Gln Asp Ser Ala Gln Ala Gly Glu Val Asp His Thr Leu
    290                 295                 300

Leu Gly Gln Cys Thr Gly Ala Gly Tyr Phe Met Gln Phe Ser Thr Ser
305                 310                 315                 320

Ser Gly Ser Ala Glu Glu Ala Ala Leu Leu Glu Ser Arg Ile Leu Tyr
                325                 330                 335

Pro Lys Arg Lys Gln Gln Cys Leu Gln Phe Phe Tyr Lys Met Thr Gly
            340                 345                 350

Ser Pro Ser Asp Arg Leu Val Val Trp Val Arg Arg Asp Asp Ser Thr
            355                 360                 365

Gly Asn Val Arg Lys Leu Val Lys Val Gln Thr Phe Gln Gly Asp Asp
            370                 375                 380

Asp His Asn Trp Lys Ile Ala His Val Val Leu Lys Glu Glu Gln Lys
385                 390                 395                 400

Phe Arg Tyr Leu Phe Gln Gly Thr Lys Gly Asp Pro Gln Asn Ser Thr
                405                 410                 415

Gly Gly Ile Tyr Leu Asp Asp Ile Thr Leu Thr Glu Thr Pro Cys Pro
```

```
                420                      425                          430


        Thr Gly Val Trp Thr Val Arg Asn Phe Ser Gln Val Leu Glu Asn Thr
                    435                 440                 445


        Ser Lys Gly Asp Lys Leu Gln Ser Pro Arg Phe Tyr Asn Ser Glu Gly
            450                 455                 460


        Tyr Gly Phe Gly Val Thr Leu Tyr Pro Asn Ser Arg Glu Ser Ser Gly
        465                 470                 475                 480


        Tyr Leu Arg Leu Ala Phe His Val Cys Ser Gly Glu Asn Asp Ala Ile
                    485                 490                 495


        Leu Glu Trp Pro Val Glu Asn Arg Gln Val Ile Ile Thr Ile Leu Asp
                    500                 505                 510


        Gln Glu Pro Asp Val Arg Asn Arg Met Ser Ser Ser Met Val Phe Thr
                515                 520                 525


        Thr Ser Lys Ser His Thr Ser Pro Ala Ile Asn Asp Thr Val Ile Trp
            530                 535                 540


        Asp Arg Pro Ser Arg Val Gly Thr Tyr His Thr Asp Cys Asn Cys Phe
        545                 550                 555                 560


        Arg Ser Ile Asp Leu Gly Trp Ser Gly Phe Ile Ser His Gln Met Leu
                    565                 570                 575


        Lys Arg Arg Ser Phe Leu Lys Asn Asp Asp Leu Ile Ile Phe Val Asp
                    580                 585                 590


        Phe Glu Asp Ile Thr His Leu Ser Gln Thr Glu Val Pro Thr Lys Gly
                    595                 600                 605


        Lys Arg Leu Ser Pro Gln Gly Leu Ile Leu Gln Gly Gln Glu Gln Gln
                    610                 615                 620


        Val Ser Glu Glu Gly Ser Gly Lys Ala Met Leu Glu Glu Ala Leu Pro
        625                 630                 635                 640


        Val Ser Leu Ser Gln Gly Gln Pro Ser Arg Gln Lys Arg Ser Val Glu
                    645                 650                 655


        Asn Thr Gly Pro Leu Glu Asp His Asn Trp Pro Gln Tyr Phe Arg Asp
                    660                 665                 670
```

```
Pro Cys Asp Pro Asn Pro Cys Gln Asn Asp Gly Ile Cys Val Asn Val
        675                 680                 685

Lys Gly Met Ala Ser Cys Arg Cys Ile Ser Gly His Ala Phe Phe Tyr
        690                 695                 700

Thr Gly Glu Arg Cys Gln Ala Val Gln Val His Gly Ser Val Leu Gly
705                 710                 715                 720

Met Val Ile Gly Gly Thr Ala Gly Val Ile Phe Leu Thr Phe Ser Ile
                725                 730                 735

Ile Ala Ile Leu Ser Gln Arg Pro Arg Lys
        740                 745
```

<210> 2
<211> 747
<212> PRT
<213> Mus musculus

<400> 2

```
Met Leu Trp Ile Gln Pro Ala Cys Leu Leu Ser Leu Ile Phe Ser Ala
1               5               10                  15

His Ile Ala Ala Val Ser Ile Lys His Leu Leu Asn Gly Ser Asp His
        20                  25                  30

Asp Thr Asp Val Gly Glu Gln Lys Asp Ile Phe Glu Ile Asn Leu Ala
        35                  40                  45

Ala Gly Leu Asn Leu Phe Gln Gly Asp Ile Leu Leu Pro Arg Thr Arg
        50                  55                  60

Asn Ala Met Arg Asp Pro Ser Ser Arg Trp Lys Leu Pro Ile Pro Tyr
65                  70                  75                  80

Ile Leu Ala Asp Asn Leu Glu Leu Asn Ala Lys Gly Ala Ile Leu His
                85                  90                  95

Ala Phe Glu Met Phe Arg Leu Lys Ser Cys Val Asp Phe Lys Pro Tyr
            100                 105                 110

Glu Gly Glu Ser Ser Tyr Ile Ile Phe Gln Lys Leu Ser Gly Cys Trp
        115                 120                 125

Ser Met Ile Gly Asp Gln Gln Val Gly Gln Asn Ile Ser Ile Gly Glu
        130                 135                 140
```

```
Gly Cys Asp Phe Lys Ala Thr Ile Glu His Glu Ile Leu His Ala Leu
145             150             155             160

Gly Phe Phe His Glu Gln Ser Arg Thr Asp Arg Asp Asp Tyr Val Asn
                165             170             175

Ile Trp Trp Asp Gln Ile Ile Thr Asp Tyr Glu His Asn Phe Asn Thr
            180             185             190

Tyr Asp Asp Asn Thr Ile Thr Asp Leu Asn Thr Pro Tyr Asp Tyr Glu
            195             200             205

Ser Leu Met His Tyr Gly Pro Phe Ser Phe Asn Lys Asn Glu Ser Ile
            210             215             220

Pro Thr Ile Thr Thr Lys Ile Pro Glu Phe Asn Thr Ile Ile Gly Gln
225             230             235             240

Leu Pro Asp Phe Ser Ala Ile Asp Leu Ile Arg Leu Asn Arg Met Tyr
                245             250             255

Asn Cys Thr Ala Thr His Thr Leu Leu Asp His Cys Asp Phe Glu Lys
            260             265             270

Thr Asn Val Cys Gly Met Ile Gln Gly Thr Arg Asp Asp Ala Asp Trp
            275             280             285

Ala His Gly Asp Ser Ser Gln Pro Glu Gln Val Asp His Thr Leu Val
            290             295             300

Gly Gln Cys Lys Gly Ala Gly Tyr Phe Met Phe Phe Asn Thr Ser Leu
305             310             315             320

Gly Ala Arg Gly Glu Ala Ala Leu Leu Glu Ser Arg Ile Leu Tyr Pro
                325             330             335

Lys Arg Lys Gln Gln Cys Leu Gln Phe Phe Tyr Lys Met Thr Gly Ser
            340             345             350

Pro Ala Asp Arg Phe Glu Val Trp Val Arg Arg Asp Asp Asn Ala Gly
            355             360             365

Lys Val Arg Gln Leu Ala Lys Ile Gln Thr Phe Gln Gly Asp Ser Asp
            370             375             380

His Asn Trp Lys Ile Ala His Val Thr Leu Asn Glu Glu Lys Lys Phe
385             390             395             400
```

```
Arg Tyr Val Phe Leu Gly Thr Lys Gly Asp Pro Gly Asn Ser Ser Gly
            405             410             415

Gly Ile Tyr Leu Asp Asp Ile Thr Leu Thr Glu Thr Pro Cys Pro Ala
            420             425             430

Gly Val Trp Thr Ile Arg Asn Ile Ser Gln Ile Leu Glu Asn Thr Val
            435             440             445

Lys Gly Asp Lys Leu Val Ser Pro Arg Phe Tyr Asn Ser Glu Gly Tyr
            450             455             460

Gly Val Gly Val Thr Leu Tyr Pro Asn Gly Arg Ile Thr Ser Asn Ser
465             470             475             480

Gly Phe Leu Gly Leu Thr Phe His Leu Tyr Ser Gly Asp Asn Asp Ala
            485             490             495

Ile Leu Glu Trp Pro Val Glu Asn Arg Gln Ala Ile Met Thr Ile Leu
            500             505             510

Asp Gln Glu Ala Asp Thr Arg Asn Arg Met Ser Leu Thr Leu Met Phe
            515             520             525

Thr Thr Ser Lys Asn Gln Thr Ser Ser Ala Ile Asn Gly Ser Val Ile
            530             535             540

Trp Asp Arg Pro Ser Lys Val Gly Val Tyr Asp Lys Asp Cys Asp Cys
545             550             555             560

Phe Arg Ser Leu Asp Trp Gly Trp Gly Gln Ala Ile Ser His Gln Leu
            565             570             575

Leu Lys Arg Arg Asn Phe Leu Lys Gly Asp Ser Leu Ile Ile Phe Val
            580             585             590

Asp Phe Lys Asp Leu Thr His Leu Asn Arg Thr Glu Val Pro Ala Ser
            595             600             605

Ala Arg Ser Thr Met Pro Arg Gly Leu Leu Leu Gln Gly Gln Glu Ser
            610             615             620

Pro Ala Leu Gly Glu Ser Ser Arg Lys Ala Met Leu Glu Glu Ser Leu
625             630             635             640

Pro Ser Ser Leu Gly Gln Arg His Pro Ser Arg Gln Lys Arg Ser Val
            645             650             655
```

```
        Glu Asn Thr Gly Pro Met Glu Asp His Asn Trp Pro Gln Tyr Phe Arg
                    660             665             670

        Asp Pro Cys Asp Pro Asn Pro Cys Gln Asn Glu Gly Thr Cys Val Asn
                    675             680             685

        Val Lys Gly Met Ala Ser Cys Arg Cys Val Ser Gly His Ala Phe Phe
                    690             695             700

        Tyr Ala Gly Glu Arg Cys Gln Ala Met His Val His Gly Ser Leu Leu
        705             710             715             720

        Gly Leu Leu Ile Gly Cys Ile Ala Gly Leu Ile Phe Leu Thr Phe Val
                        725             730             735

        Thr Phe Ser Thr Thr Asn Gly Lys Leu Arg Gln
                    740             745
```

<210> 3
<211> 748
<212> PRT
<213> Rat

<400> 3

```
        Met Leu Trp Thr Leu Pro Val Cys Leu Leu Ser Leu Ser Phe Ser Ala
        1               5               10              15

        His Ile Ala Ala Val Ser Ile Gln His Leu Ser Thr Gly His Asp His
                    20              25              30

        Asp Asp Val Asp Val Gly Glu Gln Gln Lys Asp Ile Ser Glu Ile Asn
                    35              40              45

        Ser Ala Ala Gly Leu Asn Leu Phe Gln Gly Asp Ile Leu Leu Pro Arg
                50              55              60

        Thr Arg Asn Ala Leu Arg Asp Pro Ser Ser Arg Trp Lys Leu Pro Ile
        65              70              75              80

        Pro Tyr Ile Leu Ala Asp Asn Leu Asp Leu Asn Ala Lys Gly Ala Ile
                        85              90              95

        Leu Asn Ala Phe Glu Met Phe Arg Leu Lys Ser Cys Val Asp Phe Lys
                    100             105             110

        Pro Tyr Glu Gly Glu Ser Ser Tyr Ile Ile Phe Gln Gln Phe Ser Gly
                    115             120             125
```

```
Cys Trp Ser Met Val Gly Asp Gln His Val Gly Gln Asn Ile Ser Ile
    130                 135             140

Gly Glu Gly Cys Asp Tyr Lys Ala Ile Ile Glu His Glu Ile Leu His
    145                 150             155                 160

Ala Leu Gly Phe Phe His Glu Gln Ser Arg Thr Asp Arg Asp Asp Tyr
                165             170             175

Val Asn Ile Trp Trp Asn Glu Ile Met Thr Asp Tyr Glu His Asn Phe
            180             185             190

Asn Thr Tyr Asp Asp Lys Thr Ile Thr Asp Leu Asn Thr Pro Tyr Asp
        195             200             205

Tyr Glu Ser Leu Met His Tyr Gly Pro Phe Ser Phe Asn Lys Asn Glu
    210             215             220

Thr Ile Pro Thr Ile Thr Thr Lys Ile Pro Glu Phe Asn Ala Ile Ile
225             230             235             240

Gly Gln Arg Leu Asp Phe Ser Ala Thr Asp Leu Thr Arg Leu Asn Arg
            245             250             255

Met Tyr Asn Cys Thr Arg Thr His Thr Leu Leu Asp His Cys Ala Phe
        260             265             270

Glu Lys Thr Asn Ile Cys Gly Met Ile Gln Gly Thr Arg Asp Asp Ala
    275             280             285

Asp Trp Val His Glu Asp Ser Ser Gln Pro Gly Gln Val Asp His Thr
    290             295             300

Leu Val Gly Arg Cys Lys Ala Ala Gly Tyr Phe Met Tyr Phe Asn Thr
305             310             315             320

Ser Ser Gly Val Thr Gly Glu Val Ala Leu Leu Glu Ser Arg Ile Leu
            325             330             335

Tyr Pro Lys Arg Lys Gln Gln Cys Leu Gln Phe Phe Tyr Lys Met Thr
        340             345             350

Gly Ser Pro Ala Asp Arg Leu Leu Ile Trp Val Arg Arg Asp Asp Asn
    355             360             365

Thr Gly Asn Val Cys Gln Leu Ala Lys Ile Gln Thr Phe Gln Gly Asp
```

370                     375                     380

Ser Asp His Asn Trp Lys Ile Ala His Val Thr Leu Asn Glu Glu Lys
385                390                395                400

Lys Phe Arg Tyr Val Phe Gln Gly Thr Lys Gly Asp Pro Gly Asn Ser
              405                410                415

Asp Gly Gly Ile Tyr Leu Asp Asp Ile Thr Leu Thr Glu Thr Pro Cys
          420                425                430

Pro Thr Gly Val Trp Thr Ile Arg Asn Ile Ser Gln Val Leu Glu Asn
          435                440                445

Thr Val Lys Gly Asp Arg Leu Val Ser Pro Arg Phe Tyr Asn Ser Glu
      450                455                460

Gly Tyr Gly Phe Gly Val Thr Leu Tyr Pro Asn Gly Arg Ile Thr Ser
465                470                475                480

Asn Ser Gly Tyr Leu Gly Leu Ala Phe His Leu Tyr Ser Gly Asp Asn
              485                490                495

Asp Val Ile Leu Glu Trp Pro Val Glu Asn Arg Gln Ala Ile Met Thr
          500                505                510

Ile Leu Asp Gln Glu Pro Asp Ala Arg Asn Arg Met Ser Leu Ser Leu
          515                520                525

Met Phe Thr Thr Ser Lys Tyr Gln Thr Ser Ser Ala Ile Asn Gly Ser
      530                535                540

Val Ile Trp Asp Arg Pro Thr Lys Val Gly Val Tyr Asp Lys Asp Cys
545                550                555                560

Asp Cys Phe Arg Ser Ile Asp Trp Gly Trp Gly Gln Ala Ile Ser His
              565                570                575

Gln Met Leu Met Arg Arg Asn Phe Leu Lys Asp Asp Thr Leu Ile Ile
          580                585                590

Phe Val Asp Phe Lys Asp Leu Thr His Leu Arg Gln Thr Glu Val Pro
          595                600                605

Ile Pro Ser Arg Ser Val Ile Pro Arg Gly Leu Leu Leu Gln Gly Gln
      610                615                620

26

```
Glu Pro Leu Ala Leu Gly Asp Ser Arg Ile Ala Met Met Glu Glu Ser
625                 630                 635                 640

Leu Pro Arg Arg Leu Asp Gln Arg Gln Pro Ser Arg Pro Lys Arg Ser
                    645                 650                 655

Val Glu Asn Thr Gly Pro Met Glu Asp His Asn Trp Pro Gln Tyr Phe
                660                 665                 670

Arg Asp Pro Cys Asp Pro Asn Pro Cys Gln Asn Glu Gly Thr Cys Val
            675                 680                 685

Asn Val Lys Gly Met Ala Ser Cys Arg Cys Val Ser Gly His Ala Phe
        690                 695                 700

Phe Tyr Thr Gly Glu Arg Cys Gln Ala Met His Val His Gly Ser Leu
705                 710                 715                 720

Leu Gly Leu Leu Ile Gly Cys Ile Thr Ala Leu Ile Phe Leu Thr Phe
                725                 730                 735

Ile Thr Phe Ser Asn Thr Tyr Gln Lys Leu Arg Gln
                740                 745
```

<210> 4
<211> 701
<212> PRT
<213> Human

<400> 4

```
Met Asp Leu Trp Asn Leu Ser Trp Phe Leu Phe Leu Asp Ala Leu Leu
1                   5                   10                  15

Val Ile Ser Gly Leu Ala Thr Pro Glu Asn Phe Asp Val Asp Gly Gly
                20                  25                  30

Met Asp Gln Asp Ile Phe Asp Ile Asn Glu Gly Leu Gly Leu Asp Leu
            35                  40                  45

Phe Glu Gly Asp Ile Arg Leu Asp Arg Ala Gln Ile Arg Asn Ser Ile
    50                  55                  60

Ile Gly Glu Lys Tyr Arg Trp Pro His Thr Ile Pro Tyr Val Leu Glu
65                  70                  75                  80

Asp Ser Leu Glu Met Asn Ala Lys Gly Val Ile Leu Asn Ala Phe Glu
                85                  90                  95
```

27

```
Arg Tyr Arg Leu Lys Thr Cys Ile Asp Phe Lys Pro Trp Ala Gly Glu
        100             105             110

Thr Asn Tyr Ile Ser Val Phe Lys Gly Ser Gly Cys Trp Ser Ser Val
        115             120             125

Gly Asn Arg Arg Val Gly Lys Gln Glu Leu Ser Ile Gly Ala Asn Cys
        130             135             140

Asp Arg Ile Ala Thr Val Gln His Glu Phe Leu His Ala Leu Gly Phe
145             150             155             160

Trp His Glu Gln Ser Arg Ser Asp Arg Asp Asp Tyr Val Arg Ile Met
            165             170             175

Trp Asp Arg Ile Leu Ser Gly Arg Glu His Asn Phe Asn Thr Tyr Ser
        180             185             190

Asp Asp Ile Ser Asp Ser Leu Asn Val Pro Tyr Asp Tyr Thr Ser Val
        195             200             205

Met His Tyr Ser Lys Thr Ala Phe Gln Asn Gly Thr Glu Pro Thr Ile
        210             215             220

Val Thr Arg Ile Ser Asp Phe Glu Asp Val Ile Gly Gln Arg Met Asp
225             230             235             240

Phe Ser Asp Ser Asp Leu Leu Lys Leu Asn Gln Leu Tyr Asn Cys Ser
            245             250             255

Ser Ser Leu Ser Phe Met Asp Ser Cys Ser Phe Glu Leu Glu Asn Val
            260             265             270

Cys Gly Met Ile Gln Ser Ser Gly Asp Asn Ala Asp Trp Gln Arg Val
        275             280             285

Ser Gln Val Pro Arg Gly Pro Glu Ser Asp His Ser Asn Met Gly Gln
        290             295             300

Cys Gln Gly Ser Gly Phe Phe Met His Phe Asp Ser Ser Ser Val Asn
305             310             315             320

Val Gly Ala Thr Ala Val Leu Glu Ser Arg Thr Leu Tyr Pro Lys Arg
            325             330             335

Gly Phe Gln Cys Leu Gln Phe Tyr Leu Tyr Asn Ser Gly Ser Glu Ser
        340             345             350
```

```
Asp Gln Leu Asn Ile Tyr Ile Arg Glu Tyr Ser Ala Asp Asn Val Asp
        355             360             365

Gly Asn Leu Thr Leu Val Glu Glu Ile Lys Glu Ile Pro Thr Gly Ser
        370             375             380

Trp Gln Leu Tyr His Val Thr Leu Lys Val Thr Lys Lys Phe Arg Val
385                 390             395                 400

Val Phe Glu Gly Arg Lys Gly Ser Gly Ala Ser Leu Gly Gly Leu Ser
                405             410                 415

Ile Asp Asp Ile Asn Leu Ser Glu Thr Arg Cys Pro His His Ile Trp
            420             425             430

His Ile Arg Asn Phe Thr Gln Phe Ile Gly Ser Pro Asn Gly Thr Leu
            435             440             445

Tyr Ser Pro Pro Phe Tyr Ser Ser Lys Gly Tyr Ala Phe Gln Ile Tyr
        450             455             460

Leu Asn Leu Ala His Val Thr Asn Ala Gly Ile Tyr Phe His Leu Ile
465                 470             475                 480

Ser Gly Ala Asn Asp Asp Gln Leu Gln Trp Pro Cys Pro Trp Gln Gln
                485             490                 495

Ala Thr Met Thr Leu Leu Asp Gln Asn Pro Asp Ile Arg Gln Arg Met
            500             505             510

Ser Asn Gln Arg Ser Ile Thr Thr Asp Pro Phe Met Thr Thr Asp Asn
        515             520             525

Gly Asn Tyr Phe Trp Asp Arg Pro Ser Lys Val Gly Thr Val Ala Leu
        530             535             540

Phe Ser Asn Gly Thr Gln Phe Arg Arg Gly Gly Gly Tyr Gly Thr Ser
545                 550             555                 560

Ala Phe Ile Thr His Glu Arg Leu Lys Ser Arg Asp Phe Ile Lys Gly
            565             570             575

Asp Asp Val Tyr Ile Leu Leu Thr Val Glu Asp Ile Ser His Leu Asn
            580             585             590

Ser Thr Gln Ile Gln Leu Thr Pro Ala Pro Ser Val Gln Asp Leu Cys
        595             600             605
```

```
Ser Lys Thr Thr Cys Lys Asn Asp Gly Val Cys Thr Val Arg Asp Gly
    610             615             620

Lys Ala Glu Cys Arg Cys Gln Ser Gly Glu Asp Trp Trp Tyr Met Gly
625             630             635                 640

Glu Arg Cys Glu Lys Arg Gly Ser Thr Arg Asp Thr Ile Val Ile Ala
            645             650                 655

Val Ser Ser Thr Val Ala Val Phe Ala Leu Met Leu Ile Ile Thr Leu
        660             665             670

Val Ser Val Tyr Cys Thr Arg Lys Lys Tyr Arg Glu Arg Met Ser Ser
        675             680             685

Asn Arg Pro Asn Leu Thr Pro Gln Asn Gln His Ala Phe
    690             695             700
```

<210> 5
<211> 704
<212> PRT
<213> Mus musculus

<400> 5

```
Met Asp Ala Arg His Gln Pro Trp Phe Leu Val Phe Ala Thr Phe Leu
1               5               10              15

Leu Val Ser Gly Leu Pro Ala Pro Glu Lys Phe Val Lys Asp Ile Asp
            20              25              30

Gly Gly Ile Asp Gln Asp Ile Phe Asp Ile Asn Gln Gly Leu Gly Leu
        35              40              45

Asp Leu Phe Glu Gly Asp Ile Lys Leu Glu Ala Asn Gly Lys Asn Ser
    50              55              60

Ile Ile Gly Asp His Lys Arg Trp Pro His Thr Ile Pro Tyr Val Leu
65              70              75                  80

Glu Asp Ser Leu Glu Met Asn Ala Lys Gly Val Ile Leu Asn Ala Phe
            85              90              95

Glu Arg Tyr Arg Leu Lys Thr Cys Ile Asp Phe Lys Pro Trp Ser Gly
        100             105             110

Glu Ala Asn Tyr Ile Ser Val Phe Lys Gly Ser Gly Cys Trp Ser Ser
        115             120             125
```

30

```
Val Gly Asn Ile His Ala Gly Lys Gln Glu Leu Ser Ile Gly Thr Asn
    130                 135                 140

Cys Asp Arg Ile Ala Thr Val Gln His Glu Phe Leu His Ala Leu Gly
145                 150                 155                 160

Phe Trp His Glu Gln Ser Arg Ala Asp Arg Asp Asp Tyr Val Ile Ile
                165                 170                 175

Val Trp Asp Arg Ile Gln Pro Gly Lys Glu His Asn Phe Asn Ile Tyr
                180                 185                 190

Asn Asp Ser Val Ser Asp Ser Leu Asn Val Pro Tyr Asp Tyr Thr Ser
                195                 200                 205

Val Met His Tyr Ser Lys Thr Ala Phe Gln Asn Gly Thr Glu Ser Thr
    210                 215                 220

Ile Val Thr Arg Ile Ser Glu Phe Glu Asp Val Ile Gly Gln Arg Met
225                 230                 235                 240

Asp Phe Ser Asp Tyr Asp Leu Leu Lys Leu Asn Gln Leu Tyr Asn Cys
                245                 250                 255

Thr Ser Ser Leu Ser Phe Met Asp Ser Cys Asp Phe Glu Leu Glu Asn
                260                 265                 270

Ile Cys Gly Met Ile Gln Ser Ser Gly Asp Ser Ala Asp Trp Gln Arg
                275                 280                 285

Val Ser Gln Val Leu Ser Gly Pro Glu Ser Asp His Ser Lys Met Gly
    290                 295                 300

Gln Cys Lys Asp Ser Gly Phe Phe Met His Phe Asn Thr Ser Ile Leu
305                 310                 315                 320

Asn Glu Gly Ala Thr Ala Met Leu Glu Ser Arg Leu Leu Tyr Pro Lys
                325                 330                 335

Arg Gly Phe Gln Cys Leu Glu Phe Tyr Leu Tyr Asn Ser Gly Ser Gly
                340                 345                 350

Asn Asp Gln Leu Asn Ile Tyr Thr Arg Glu Tyr Thr Thr Gly Gln Gln
                355                 360                 365

Gly Gly Val Leu Thr Leu Gln Arg Gln Ile Lys Glu Val Pro Ile Gly
```

```
            370                      375                        380

Ser Trp Gln Leu His Tyr Val Thr Leu Gln Val Thr Lys Lys Phe Arg
385                 390                 395                 400

Val Val Phe Glu Gly Leu Arg Gly Pro Gly Thr Ser Ser Gly Gly Leu
                405                 410                 415

Ser Ile Asp Asp Ile Asn Leu Ser Glu Thr Arg Cys Pro His His Ile
            420                 425                 430

Trp His Ile Gln Asn Phe Thr Gln Ile Leu Gly Gly Gln Asp Thr Ser
        435                 440                 445

Val Tyr Ser Pro Pro Phe Tyr Ser Ser Lys Gly Tyr Ala Phe Gln Ile
    450                 455                 460

Tyr Met Asp Leu Arg Ser Ser Thr Asn Val Gly Ile Tyr Phe His Leu
465                 470                 475                 480

Ile Ser Gly Ala Asn Asp Asp Gln Leu Gln Trp Pro Cys Pro Trp Gln
            485                 490                 495

Gln Ala Thr Met Thr Leu Leu Asp Gln Asn Pro Asp Ile Arg Gln Arg
            500                 505                 510

Met Phe Asn Gln Arg Ser Ile Thr Thr Asp Pro Thr Met Thr Ser Asp
        515                 520                 525

Asn Gly Ser Tyr Phe Trp Asp Arg Pro Ser Lys Val Gly Val Thr Asp
        530                 535                 540

Val Phe Pro Asn Gly Thr Gln Phe Ser Arg Gly Ile Gly Tyr Gly Thr
545                 550                 555                 560

Thr Val Phe Ile Thr Arg Glu Arg Leu Lys Ser Arg Glu Phe Ile Lys
                565                 570                 575

Gly Asp Asp Ile Tyr Ile Leu Leu Thr Val Glu Asp Ile Ser His Leu
            580                 585                 590

Asn Ser Thr Ser Ala Val Pro Asp Pro Val Pro Thr Leu Ala Val His
        595                 600                 605

Asn Ala Cys Ser Glu Val Val Cys Gln Asn Gly Gly Ile Cys Val Val
    610                 615                 620
```

```
Gln Asp Gly Arg Ala Glu Cys Lys Cys Pro Ala Gly Glu Asp Trp Trp
625                 630             635                 640

Tyr Met Gly Lys Arg Cys Glu Lys Arg Gly Ser Thr Arg Asp Thr Val
                645             650                 655

Ile Ile Ala Val Ser Ser Thr Val Thr Val Phe Ala Val Met Leu Ile
            660             665                 670

Ile Thr Leu Val Ser Val Tyr Cys Thr Arg Arg Lys Tyr Arg Lys Lys
            675             680                 685

Ala Arg Ala Asn Thr Ala Ala Met Thr Leu Glu Asn Gln His Ala Phe
        690             695             700
```

<210> 6
<211> 704
<212> PRT
<213> Rat

<400> 6

```
Met Asp Ala Arg His Trp Pro Trp Phe Leu Val Phe Ala Thr Phe Leu
1               5               10                  15

Leu Val Ser Gly Leu Pro Ala Pro Glu Lys Phe Val Lys Asp Ile Asp
            20              25                  30

Gly Gly Ile Asp Gln Asp Ile Phe Asp Ile Asn Glu Asp Leu Gly Leu
            35              40                  45

Asp Leu Phe Glu Gly Asp Ile Lys Leu Glu Ala Ser Gly Arg Asn Ser
    50              55              60

Ile Ile Gly Asp Asn Tyr Arg Trp Pro His Thr Ile Pro Tyr Val Leu
65              70              75                  80

Glu Asp Ser Leu Glu Met Asn Ala Lys Gly Val Ile Leu Asn Ala Phe
            85              90                  95

Glu Arg Tyr Arg Leu Lys Thr Cys Ile Asp Phe Lys Pro Trp Ser Gly
            100             105                 110

Glu Glu Asn Tyr Ile Ser Val Phe Lys Gly Ser Gly Cys Trp Ser Ser
            115             120             125

Val Gly Asn Ile His Ala Gly Lys Gln Glu Leu Ser Ile Gly Thr Asn
    130             135             140
```

```
Cys Asp Arg Ile Ala Thr Val Gln His Glu Phe Leu His Ala Leu Gly
145                 150             155                 160

Phe Trp His Glu Gln Ser Arg Ala Asp Arg Asp Asp Tyr Ile Thr Ile
                165             170             175

Val Trp Asp Arg Ile Leu Ser Gly Lys Glu His Asn Phe Asn Ile Tyr
            180             185             190

Asn Asp Ser Val Ser Asp Ser Leu Asn Val Pro Tyr Asp Tyr Thr Ser
            195             200             205

Val Met His Tyr Ser Lys Thr Ala Phe Gln Asn Gly Thr Glu Ser Thr
            210             215             220

Ile Ile Thr Lys Ile Ser Asp Phe Glu Asp Val Ile Gly Gln Arg Met
225             230             235             240

Asp Phe Ser Asp Tyr Asp Leu Leu Lys Leu Asn Gln Leu Tyr Ser Cys
                245             250             255

Thr Ser Ser Leu Ser Phe Met Asp Ser Cys Asp Phe Glu Leu Glu Asn
            260             265             270

Ile Cys Gly Met Ile Gln Ser Ser Gln Asp Ser Ala Asp Trp Gln Arg
            275             280             285

Leu Ser Gln Val Leu Ser Gly Pro Glu Asn Asp His Ser Asn Met Gly
            290             295             300

Gln Cys Lys Asp Ser Gly Phe Phe Met His Phe Asn Thr Ser Thr Gly
305             310             315             320

Asn Gly Gly Val Thr Ala Met Leu Glu Ser Arg Val Leu Tyr Pro Lys
                325             330             335

Arg Gly Phe Gln Cys Val Glu Phe Tyr Leu Tyr Asn Ser Gly Ser Gly
                340             345             350

Asn Gly Gln Leu Asn Val Tyr Thr Arg Glu Tyr Thr Ala Gly His Gln
            355             360             365

Asp Gly Val Leu Thr Leu Gln Arg Glu Ile Arg Asp Ile Pro Thr Gly
            370             375             380

Ser Trp Gln Leu Tyr Tyr Val Thr Leu Gln Val Thr Glu Lys Phe Arg
385             390             395             400
```

34

```
Val Val Phe Glu Gly Val Gly Gly Pro Gly Ala Ser Ser Gly Gly Leu
            405                 410                 415

Ser Ile Asp Asp Ile Asn Leu Ser Glu Thr Arg Cys Pro His His Ile
            420                 425                 430

Trp His Ile Gln Asn Phe Thr Gln Leu Leu Gly Gly Gln Thr Thr Val
            435                 440                 445

Tyr Ser Pro Pro Phe Tyr Ser Ser Lys Gly Tyr Ala Phe Gln Ile Asn
        450                 455                 460

Leu Asp Leu Thr Ser Pro Thr Asn Val Gly Leu Tyr Phe His Leu Ile
465                 470                 475                 480

Ser Gly Ala Asn Asp Asp Gln Leu Gln Trp Pro Cys Pro Trp Gln Gln
            485                 490                 495

Ala Thr Met Thr Leu Leu Asp Gln Asn Pro Asp Ile Arg Gln Arg Met
            500                 505                 510

Ser Asn Gln Arg Ser Ile Thr Thr Asp Pro Lys Met Thr Asp Asp Asn
            515                 520                 525

Gly Ser Tyr Leu Trp Asp Arg Pro Ser Lys Val Gly Val Glu Ala Phe
            530                 535                 540

Phe Pro Asn Gly Thr Gln Phe Ser Arg Gly Arg Gly Tyr Gly Thr Ser
545                 550                 555                 560

Val Phe Ile Thr Gln Glu Arg Leu Lys Ser Arg Glu Phe Leu Lys Gly
            565                 570                 575

Asp Asp Val Tyr Ile Leu Leu Thr Val Glu Asp Ile Ser His Leu Asn
            580                 585                 590

Ser Thr Ala Ala Val Pro Gly Pro Val Pro Thr Thr Ser Thr Val His
            595                 600                 605

Asn Ala Cys Ser Glu Val Glu Cys Gln Asn Gly Gly Ile Cys Thr Leu
            610                 615                 620

Gln Glu Gly Arg Ala Glu Cys Lys Cys Pro Ala Gly Glu Asp Trp Trp
625                 630                 635                 640

Tyr Met Gly Lys Arg Cys Glu Lys Arg Gly Ser Thr Lys Asp Thr Ile
                645                 650                 655
```

```
Val Ile Ala Val Ser Ser Thr Val Thr Val Phe Ala Val Met Leu Ile
        660             665             670

Ile Thr Leu Ile Ser Val Tyr Cys Thr Arg Arg Lys Tyr Arg Lys Lys
        675             680             685

Ala Ser Ala Lys Thr Ala Ala Met Asn Leu Glu Asn Gln His Ala Phe
        690             695             700
```

<210> 7
<211> 732
<212> PRT
<213> Porphyromonas gingivalis

<400> 7

```
Met Lys Lys Thr Ile Phe Gln Gln Leu Phe Leu Ser Val Cys Ala Leu
1               5               10              15

Thr Val Ala Leu Pro Cys Ser Ala Gln Ser Pro Glu Thr Ser Gly Lys
            20              25              30

Glu Phe Thr Leu Glu Gln Leu Met Pro Gly Gly Lys Glu Phe Tyr Asn
        35              40              45

Phe Tyr Pro Glu Tyr Val Val Gly Leu Gln Trp Met Gly Asp Asn Tyr
    50              55              60

Val Phe Ile Glu Gly Asp Asp Leu Val Phe Asn Lys Ala Asn Gly Lys
65              70              75              80

Ser Ala Gln Thr Thr Arg Phe Ser Ala Ala Asp Leu Asn Ala Leu Met
                85              90              95

Pro Glu Gly Cys Lys Phe Gln Thr Thr Asp Ala Phe Pro Ser Phe Arg
            100             105             110

Thr Leu Asp Ala Gly Arg Gly Leu Val Val Leu Phe Thr Gln Gly Gly
            115             120             125

Leu Val Gly Phe Asp Met Leu Ala Arg Lys Val Thr Tyr Leu Phe Asp
    130             135             140

Thr Asn Glu Glu Thr Ala Ser Leu Asp Phe Ser Pro Val Gly Asp Arg
145             150             155             160

Val Ala Tyr Val Arg Asn His Asn Leu Tyr Ile Ala Arg Gly Gly Lys
                165             170             175
```

```
Leu Gly Glu Gly Met Ser Arg Ala Ile Ala Val Thr Ile Asp Gly Thr
        180                     185                 190

Glu Thr Leu Val Tyr Gly Gln Ala Val His Gln Arg Glu Phe Gly Ile
        195                     200                 205

Glu Lys Gly Thr Phe Trp Ser Pro Lys Gly Ser Cys Leu Ala Phe Tyr
    210                     215                 220

Arg Met Asp Gln Ser Met Val Lys Pro Thr Pro Ile Val Asp Tyr His
225                 230                 235                 240

Pro Leu Glu Ala Glu Ser Lys Pro Leu Tyr Tyr Pro Met Ala Gly Thr
                245                 250                 255

Pro Ser His His Val Thr Val Gly Ile Tyr His Leu Ala Thr Gly Lys
            260                 265                 270

Thr Val Tyr Leu Gln Thr Gly Glu Pro Lys Glu Lys Phe Leu Thr Asn
        275                 280                 285

Leu Ser Trp Ser Pro Asp Glu Asn Ile Leu Tyr Val Ala Glu Val Asn
    290                 295                 300

Arg Ala Gln Asn Glu Cys Lys Val Asn Ala Tyr Asp Ala Glu Thr Gly
305                 310                 315                 320

Arg Phe Val Arg Thr Leu Phe Val Glu Thr Asp Lys His Tyr Val Glu
            325                 330                 335

Pro Leu His Pro Leu Thr Phe Leu Pro Gly Ser Asn Asn Gln Phe Ile
        340                 345                 350

Trp Gln Ser Arg Arg Asp Gly Trp Asn His Leu Tyr Leu Tyr Asp Thr
        355                 360                 365

Thr Gly Arg Leu Ile Arg Gln Val Thr Lys Gly Glu Trp Glu Val Thr
        370                 375                 380

Asn Phe Ala Gly Phe Asp Pro Lys Gly Thr Arg Leu Tyr Phe Glu Ser
385                 390                 395                 400

Thr Glu Ala Ser Pro Leu Glu Arg His Phe Tyr Cys Ile Asp Ile Lys
                405                 410                 415

Gly Gly Lys Thr Lys Asp Leu Thr Pro Glu Ser Gly Met His Arg Thr
```

37

420 425 430

Gln Leu Ser Pro Asp Gly Ser Ala Ile Ile Asp Ile Phe Gln Ser Pro
435 440 445

Thr Val Pro Arg Lys Val Thr Val Thr Asn Ile Gly Lys Gly Ser His
450 455 460

Thr Leu Leu Glu Ala Lys Asn Pro Asp Thr Gly Tyr Ala Met Pro Glu
465 470 475 480

Ile Arg Thr Gly Thr Ile Met Ala Ala Asp Gly Gln Thr Pro Leu Tyr
485 490 495

Tyr Lys Leu Thr Met Pro Leu His Phe Asp Pro Ala Lys Lys Tyr Pro
500 505 510

Val Ile Val Tyr Val Tyr Gly Gly Pro His Ala Gln Leu Val Thr Lys
515 520 525

Thr Trp Arg Ser Ser Val Gly Gly Trp Asp Ile Tyr Met Ala Gln Lys
530 535 540

Gly Tyr Ala Val Phe Thr Val Asp Ser Arg Gly Ser Ala Asn Arg Gly
545 550 555 560

Ala Ala Phe Glu Gln Val Ile His Arg Arg Leu Gly Gln Thr Glu Met
565 570 575

Ala Asp Gln Met Cys Gly Val Asp Phe Leu Lys Ser Gln Ser Trp Val
580 585 590

Asp Ala Asp Arg Ile Gly Val His Gly Trp Ser Tyr Gly Gly Phe Met
595 600 605

Thr Thr Asn Leu Met Leu Thr His Gly Asp Val Phe Lys Val Gly Val
610 615 620

Ala Gly Gly Pro Val Ile Asp Trp Asn Arg Tyr Glu Ile Met Tyr Gly
625 630 635 640

Glu Arg Tyr Phe Asp Ala Pro Gln Glu Asn Pro Glu Gly Tyr Asp Ala
645 650 655

Ala Asn Leu Leu Lys Arg Ala Gly Asp Leu Lys Gly Arg Leu Met Leu
660 665 670

```
Ile His Gly Ala Ile Asp Pro Val Val Val Trp Gln His Ser Leu Leu
        675                 680                 685

Phe Leu Asp Ala Cys Val Lys Ala Arg Thr Tyr Pro Asp Tyr Tyr Val
        690                 695                 700

Tyr Pro Ser His Glu His Asn Val Met Gly Pro Asp Arg Val His Leu
    705                 710                 715                 720

Tyr Glu Thr Ile Thr Arg Tyr Phe Thr Asp His Leu
                725                 730
```

<210> 8
<211> 11
<212> PRT
<213> Artificial sequence

<220>
<223> Synthetic oligopeptide

<220>
<221> MOD_RES
<222> (1)..(1)
<223> BLOCKED

<220>
<221> MISC_FEATURE
<222> (9)..(9)
<223> modified by Dnp (dinitrophenyl)

<400> 8

```
Met Gly Trp Met Asp Glu Ile Asp Lys Ser Gly
1                 5                 10
```

<210> 9
<211> 8
<212> PRT
<213> Artificial sequence

<220>
<223> Synthetic oligopeptide

<220>
<221> MOD_RES
<222> (1)..(1)
<223> BLOCKED

<220>
<221> MISC_FEATURE
<222> (7)..(7)
<223> modified by Dnp (dinitrophenyl)

<400> 9

```
Tyr Val Ala Glu Ala Pro Lys Gly

                         1           5
```

<210> 10
<211> 9
<212> PRT
<213> Artificial Sequence

<220>
<223> Synthetic oligopeptide

<400> 10

```
                    Lys Lys Gly Tyr Val Ala Asp Ala Pro
                    1               5
```

<210> 11
<211> 9
<212> PRT
<213> Artificial sequence

<220>
<223> Synthetic oligopeptide

<220>
<221> MISC_FEATURE
<222> (9)..(9)
<223> modified with pNA (para-nitroaniline)

<400> 11

```
                    Lys Lys Gly Tyr Val Ala Asp Ala Pro
                    1               5
```

<210> 12
<211> 9
<212> PRT
<213> Artificial sequence

<220>
<223> Synthetic oligopeptide

<220>
<221> MISC_FEATURE
<222> (9)..(9)
<223> modified by AMC (7-amido-4-methylcoumarin)

<400> 12

```
                    Lys Lys Gly Tyr Val Ala Asp Ala Pro
                    1               5
```

<210> 13
<211> 10
<212> PRT

<213> Artificial sequence

<220>
<223> Synthetic oligopeptide

<220>
<221> MOD_RES
<222> (1)..(1)
<223> ACETYLATION

<220>
<221> MISC_FEATURE
<222> (10)..(10)
<223> modified by pNA (para-nitroaniline)

<400> 13

```
            Arg Gly Arg Gly Tyr Val Ala Asp Ala Pro
            1               5                   10
```

<210> 14
<211> 9
<212> PRT
<213> Artificial sequence

<220>
<223> Synthetic oligopeptide

<220>
<221> MOD_RES
<222> (1)..(1)
<223> ACETYLATION

<220>
<221> MISC_FEATURE
<222> (9)..(9)
<223> modified with pNA (para-nitroaniline)

<400> 14

```
            Lys Lys Gly Tyr Val Ala Asp Ala Pro
            1               5
```

<210> 15
<211> 9
<212> PRT
<213> Artificial sequence

<220>
<223> Synthetic oligipeptide

<220>
<221> MOD_RES
<222> (1)..(1)
<223> ACETYLATION

<220>
<221> MISC_FEATURE

<222> (9)..(9)
<223> modified by AMC (aminomethylcoumarin)

<400> 15

```
                    Lys Lys Gly Tyr Val Ala Asp Ala Pro
                    1               5
```

<210> 16
<211> 8
<212> PRT
<213> Artificial sequence

<220>
<223> Synthetic oligopeptide

<220>
<221> MOD_RES
<222> (1)..(1)
<223> BLOCKED

<220>
<221> MISC_FEATURE
<222> (7).. (7)
<223> modified by Dnp (dinitrophenyl)

<400> 16

```
                    Tyr Val Ala Asp Ala Pro Lys Gly
                    1               5
```

**Description**

**Field of the Invention**

The subject matter described and claimed herein relates to assays for measuring meprin activity. More particularly, the assays concern rapid, sensitive, reliable and robust assays for measuring meprin activity comprising a coupled enzyme assay suitable for high-throughput screening. In particular, the invention provides methods for 1) for assaying meprin catalytic activity; 2) identifying inhibitors of meprin activity; 3) methods for measuring the IC50 of a test compound inhibiting meprin activity; 4) methods for differentiating a competitive inhibitor of meprin from a non-competitive inhibitor of meprin; and 5) methods for identifying specific inhibitors for meprin $\alpha$ and meprin $\beta$. Further provided is a kit, which can be used to perform the methods of the invention.

**Background of the invention**

Meprins are zinc-dependent, membrane-bound proteases and members of the "astacin family" of metalloproteinases (Bond and Beynon, Protein Sci. 4: 1247-1261 (1995)). The enzymes are multidomain, oligomeric proteins. The expression is highly regulated on the transcriptional and translational level. Typically, the proteins are targeted to apical membranes of polarized epithelial cells (Eldering et al., Eur. J. Biochem. 247: 920-932 (1997)). Meprins have been identified in leukocytes, cancer cells and intestine and kidney.

Meprins have been implicated in kidney fibrosis, injury, and end-stage kidney disease (Ricardo et al., Am. J. Physiol. 270:F669-676 (1996); Sampson et al., J. Biol. Chem. 276: 34128-34188 (2001); Trachtmann et al., Biochem. Biophys. Res. Commun. 208: 498-505 (1995)).

The amino acid sequences of meprin $\alpha$ and $\beta$ are 42% identical and the domain structures are similar (Jiang et al., J. Biol. Chem. 267: 9185-9193 (1992)). Meprin-$\alpha$ and -$\beta$ form homo- or heterodimers. Due to an insertion of a domain in meprin-$\alpha$, it can undergo cleavage and might be secreted, whereas meprin-$\beta$ remains an integral type 1 transmembrane protein. Homooligomers of meprin-$\beta$ (meprin B) are primarily membrane-bound dimers. Meprin A is any isoform containing the meprin-$\alpha$ subunit.

A major difference between both enzymes is the so called 'inserted' domain in meprin $\alpha$, which includes a furin cleavage site. This results in the loss of the EGF-like transmembrane and cytosolic domains, and to subsequent release of the enzyme into the extracellular environment. Meprin $\alpha$ further oligomerizes non-covalently, building huge complexes in the mega-Dalton range. This makes it the largest secreted protease known to date.

Meprin $\alpha$ and meprin $\beta$ are putatively involved in the progression of neurodegenerative diseases. In detail, upcoming evidence strengthens the hypothesis of meprin $\beta$ being an alternative $\beta$-secretase to generate amyloid $\beta$ peptides *in vivo,* thus playing a critical role in Alzheimer disease (Bien, J., Jefferson, T., Causevic, M., Jumpertz, T., Munter, L., Multhaup, G., Weggen, S., Becker-Pauly, C., Pietrzik, C. U., The Journal of biological chemistry. 2012, 287 (40), 33304-33313, 10.1074/jbc.M112.395608; Becker-Pauly, C., Pietrzik, C. U., Frontiers in molecular neuroscience. 2016, 9, 159, 10.3389/fnmol.2016.00159). Furthermore, upcoming research results proof an involvement of meprins as procollagen proteinases as well as in inflammatory processes and intestinal immune response (Broder, C., Becker-Pauly, C., The Biochemical journal. 2013, 450 (2), 253-264, 10.1042/BJ20121751; Schütte, A., Ermund, A., Becker-Pauly, C., Johansson, M. E. V., Rodriguez-Pineiro, A. M., Backhed, F., Muller, S., Lottaz, D., Bond, J. S., Hansson, G. C., Proceedings of the National Academy of Sciences of the United States of America. 2014, 111 (34), 12396-12401, 10.1073/pnas.1407597111; Prox, J., Arnold, P., Becker-Pauly, C., Matrix biology journal of the International Society for Matrix Biology. 2015, 44-46, 7-13, 10.1016/j.matbio.2015.01.010). Therefore, the enzymes are of high interest for the development of new therapeutic drugs.

Meprin $\alpha$ and meprin $\beta$ preferentially cleave substrates with acidic amino acids in P1'-postion (Becker-Pauly, C., Barre, O., Schilling, O., dem Keller, U. auf, Ohler, A., Broder, C., Schütte, A., Kappelhoff, R., Stocker, W., Overall, C. M., Molecular & cellular proteomics MCP. 2011, 10 (9), 9233, 10.1074/mcp.M111.009233). Until now, the proteolytic degradation of the fluorogenic substrates OCK+ Abz-MGWMDEIDK(Dnp)SG-OH and S2 Abz-YVAEAPK(Dnp)G-OH has been observed to identify specific and potent inhibitors for meprins (Bertenshaw, G. P., Villa, J. P., Hengst, J. A., Bond, J. S., Biological chemistry. 2002, 383 (7-8), 1175-1183, 10.1515/BC.2002.129; Schlenzig, D., Wermann, M., Ramsbeck, D., Moenke-Wedler, T., Schilling, S., Protein expression and purification. 2015, 116, 75-81, 10.1016/j.pep.2015.08.001). Since cleavage of the internally quenched substrates by meprin $\beta$ show substrate inhibition, development of new substrates or assay conditions are essential.

**Summary of the invention**

It is thus the object of the invention to the provide assays for measuring meprin activity, which are rapid, sensitive, reliable and robust and which are suitable for high-throughput screening. The object of the invention is solved by the assay according to claim 1. The invention provides an improved catalytic assay, using a chromogenic substrate in combination with an auxiliary enzyme to further increase specificity. The chromogenic substrate is usually a labeled substrate, such as a labeled peptide. In a first, rate-determining step, the cleavage of N-terminal amino acids from the labeled peptide by a meprin occurs. Subsequently, the label is released by the auxiliary enzyme. Both steps display a consistent assay to determine meprin activity. The specificity and accuracy of the coupled assay was further evaluated by comparison of inhibitor constants of actinonin, the most potent inhibitor of meprins (Madoux, F., Tredup, C., Spicer, T. P., Scampavia, L., Chase, P. S., Hodder, P. S., Fields, G. B., Becker-Pauly, C., Minond, D., Biopolymers. 2014, 102 (5), 396-406, 10.1002/bip.22527).

This assay offers a novel tool to screen for inhibitors independent of fluorogenic substrates, leaving the opportunity to test inhibitors with intercalating or artifactual properties. Developing selective inhibitors are of high interest to validate meprins as important therapeutic targets.

Accordingly, the invention further provides a method for identifying inhibitors of meprin activity.

The invention also relates to methods for measuring the IC50 value of a test compound, which is capable of inhibiting meprin activity.

In a further embodiment, the invention provides methods for differentiating a competitive inhibitor of meprin from a non-competitive inhibitor of meprin.

As two different forms of meprin are known, meprin $\alpha$ and meprin $\beta$, it is desirable to provide inhibitors that are either specific for meprin $\alpha$ or for meprin $\beta$. In a further embodiment, the invention thus provides methods for identifying inhibitors that are specifically inhibiting either meprin $\alpha$ or meprin $\beta$.

In another embodiment, the description provides a kit which is suitable to perform the methods of the invention.

**Brief description of the drawings**

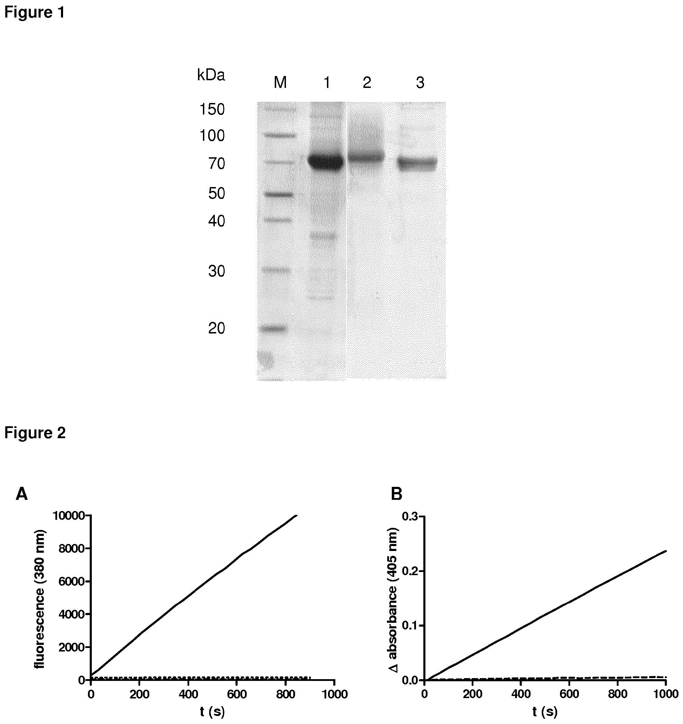

**Figure 1**    shows the SDS-PAGE analysis of the purified enzymes PtP, meprin $\beta$ and meprin $\alpha$. Lanes: M - molecular mass standards, 1 PtP, 2 meprin $\beta$ and lane 3 meprin $\alpha$. A concentration of 10 $\mu$g of each protein was applied per lane.

**Figure 2**    shows the time course of the conversion of (A) KKGYVADAP-AMC and (B) KKGYVADAP-pNA by the auxiliary enzyme PtP and meprin $\beta$ (solid line). Negative controls were taken in the presence of PtP without meprin $\beta$ and *vice versa* (dashed lines). Assay conditions were as follows: 20 mM Tris-HCl; pH 7.5, 30°C. Substrate concentrations were 0.5 M.

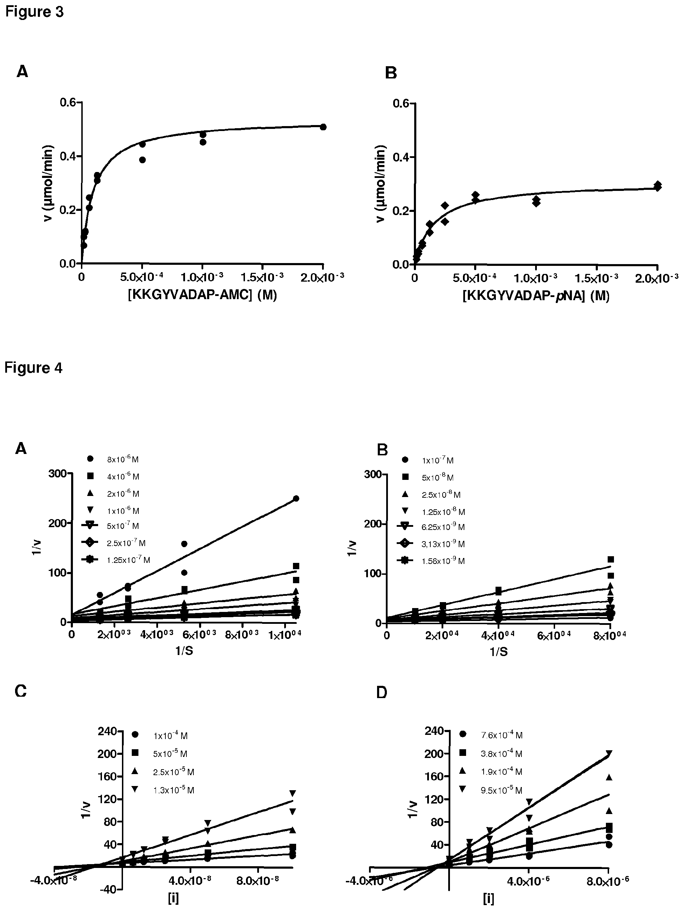

**Figure 3**    shows V/S-characteristics of the conversion of (A) the fluorogenic substrate KKGYVADAP-AMC and (B) the chromogenic substrate KKGYVADAP-pNA by the auxiliary enzyme PtP and meprin $\beta$. Measurements were performed under assay conditions. The kinetic parameters are given in Table 1.

**Figure 4**    shows Lineweaver-Burk diagrams of the conversion of KKGYVADAP-pNA by PtP and (A) meprin $\beta$ and (B) meprin $\alpha$ at different concentrations of the meprin inhibitor actinonin. Dixon plot for the cleavage of KKGYVADAP-pNA by PtP and (C) meprin $\alpha$ and (D) meprin $\beta$ in the presence of actinonin at four substrate concentration (symbols).

**Detailed description of the invention**

**Definitions**

The terms "a" and "an" mean "one or more" when used in this application, including the claims.

The term "meprin" is not limited to enzyme derived from a particular species. Thus, it is intended that all meprin orthologs are encompassed by the term meprin, including human meprin $\alpha$ and meprin $\beta$ enzymes and rodent, such as rat or mouse, meprin enzymes. The term also encompasses fragments of meprin that exhibit catalytic activity, such as the ability to cleave substrates with acidic amino acids in P1'-position. The term meprin also encompasses meprin sequences comprising one or more mutations in the amino acid sequence. Such mutations may be naturally occurring or may be engineered using standard mutagenesis techniques known in the art. A preferred meprin $\alpha$ according to the invention is meprin $\alpha$ from human (SEQ ID NO. 1), mouse (SEQ ID NO. 2) and rat (SEQ ID NO. 3) or an analogue thereof having at least 50% / 75% sequence identity / similarity, preferably 70% / 85% sequence identity / similarity, most preferably 90% / 95% sequence identity / similarity. Most preferred is human meprin $\alpha$ (SEQ ID NO. 1) or an analogue thereof having at least 50% / 75% sequence identity / similarity, preferably 70% / 85% sequence identity / similarity, most preferably 90% / 95% sequence identity / similarity. A preferred meprin $\beta$ according to the invention is meprin $\beta$ from human (SEQ ID NO. 4), mouse (SEQ ID NO. 5) and rat (SEQ ID NO. 6) or an analogue thereof having at least 50% / 75% sequence identity / similarity, preferably 70% / 85% sequence identity / similarity, most preferably 90% / 95% sequence identity / similarity. Most preferred is human meprin β (SEQ ID NO. 4) or an analogue thereof having at least 50% / 75% sequence identity / similarity, preferably 70% / 85% sequence identity / similarity, most preferably 90% / 95% sequence identity / similarity.

Meprins are zinc-dependent, membrane-bound proteases and members of the astacin family of metalloproteinases. Generally, the assay is also suitable for assaying the enzyme activity of other members of the astacin family. In the enzyme assays and screening methods described for meprin herein can thus also be employed for other members of the astacin family.

The term "PtP" means the enzyme prolyl tripeptidyl aminopeptidase. The term encompasses all forms of PtP. Further, the term PtP is not limited to enzyme derived from a particular species. Thus, it is intended that all PtP orthologs are encompassed by the term PtP. The term also encompasses fragments of PtP that exhibit catalytic activity, namely the ability to release an X-X-P tripeptide from substrates consisting of a free N-terminus and a proline in the third (C-terminal) position. Thus the term includes segments of PtP that are shorter than full-length PtP, but which still retain catalytic activity. The term PtP also encompasses PtP sequences comprising one or more mutations in the amino acid sequence. Such mutations may be naturally occurring or may be engineered using standard mutagenesis techniques known in the art. Preferred for use in the methods of the invention is PtP from microorganisms, more preferably from a bacterial PtP. Most preferred for use in the methods of invention is PtP from *Porphyromonas gingivalis* (EC:3.4.14.12, SEQ ID NO. 7).

The term "high-throughput screening" means any method or operation by which a plurality of test samples are analyzed for one or more properties, such as modulation, in particular inhibition, of meprin activity. A high-throughput operation is preferably automated.

The term "modulator" and derivatives thereof means the ability of a compound to alter the function of a meprin. The alteration may be enhancement, diminishment, activation and/or inactivation of meprin activity. Exemplary modulators of meprin comprise, but are not limited to, compounds that activate or inhibit the enzymatic activity of meprin. Preferred according to the invention are compounds that inhibit the activity of meprin, i.e. meprin inhibitors.

The term "test compound" means an agent known or suspected to interact with a meprin polypeptide or a fragment thereof. Representative test compounds include but are not restricted to, activators and inhibitors of meprin, which can e.g. be selected from peptides, enzyme substrates, co-factors, lectins, sugars, oligonucleotides, proteins, small molecules and antibodies including monoclonal antibodies, and fragments thereof such as ScFV and Fv fragments, domain antibodies, Fab fragments, and the like. Preferred according to the invention are small molecule inhibitors of meprin. An example of a small molecule inhibitor of meprin is actinonin.

**Potency of meprin inhibition**

In light of the correlation with meprin inhibition, in preferred embodiments, the subject methods utilize an agent with a Ki for meprin inhibition of 10 μM or less, more preferably of 1 μM or less, even more preferably of 0.1 μM or less or 0.01 μM or less, or most preferably 0.001 μM or less. Indeed, inhibitors with Ki values in the lower micromolar, preferably the nanomolar and even more preferably the picomolar range are contemplated.

**Molecular weight of meprin inhibitors**

In general, the most preferred meprin inhibitors of the subject methods will be small molecules, e.g., with molecular weights of 1000 g/mole or less, 500 g/mole or less, preferably of 400 g/mole or less, and even more preferably of 350 g/mole or less and even of 300 g/mole or less.

**Preparation of the meprin and PtP enzymes**

The meprin and PtP enzymes used in the assays described herein may be obtained from commercial sources or they may be prepared using the methods described herein or those known to the skilled artisan. Meprins may be isolated from any suitable animal source, for example from mammalian (e.g., rat) or chicken brains, livers, mammalian or chicken adipose tissue, mammalian or chicken skeletal muscle and/or mammalian or chicken heart muscle. and PtP may be isolated from any suitable source microorganism, for example from bacteria such as *Porphyromonas gingivalis.* Meprins and PtP may be isolated from a biological sample using standard protein purification methodology known to those of the art (see, e.g., Janson, Protein Purification: Principles, High Resolution Methods, and Applications, (2nd ed.) Wiley, New York, (1997); Rosenberg, Protein Analysis and Purification: Benchtop Techniques, Birkhauser, Boston, (1996); Walker, The Protein Protocols Handbook, Humana Press, Totowa, New Jersey, (1996); Doonan, Protein Purification Protocols, Humana Press, Totowa, New Jersey, (1996); Scopes, Protein Purification: Principles and Practice, Springer-Verlag, New York, (1994); Harris, Protein Purification Methods: A Practical Approach, IRL Press, New York, (1989)). Guidance in the isolation of an meprin and/or PtP is provided herein, (see Working Examples) Other methods for purifying active enzymes may be known to those of skill in the art and any such methods may be employed.

In a preferred embodiment of the invention, it is desired to recombinantly express the enzymes described herein. Conventional molecular biology, microbiology, recombinant DNA, and protein chemistry techniques known to those of ordinary skill of the art may be employed to produce a DNA sequence encoding an enzyme to be used in the instant assay(s). Such techniques are explained in the literature (see, e.g., Sambrook et ah, Molecular Cloning: A Laboratory Manual, (3rd ed.) Cold Spring Harbor Laboratory Press, Cold Spring Harbor, New York, USA (2001); Glover, DNA Cloning: A Practical Approach, (2nd ed.) IRL Press, New York, USA (1995); Gait, Oligonucleotide Synthesis: A Practical Approach, IRL Press, New York, USA (1984); Hames & Higgins, Nucleic Acid Hybridisation: A Practical Approach, IRL Press, Washington, D. C, USA (1985); Hames & Higgins, Protein Expression: A Practical Approach, Oxford University Press, New York, USA, (1999); Masters, Animal Cell Culture: A Practical Approach, Oxford University Press, New York, USA (2000); Bickerstaff, Immobilization of Cells And Enzymes, Humana Press, Totowa, New Jersey, USA (1997); Perbal, A Practical Guide To Molecular Cloning (2nd ed.) Wiley, New York, New York, USA (1988); Current Protocols in Molecular Biology, (Ausubel et ah, eds.), Greene Publishing Associates and Wiley-Interscience, New York (2002); Ausubel, Short Protocols in Molecular Biology: A Compendium of Methods from Current Protocols in Molecular Biology, (4th ed.) John Wiley & Sons, New York, New York, USA (1999)).

A DNA sequence encoding a meprin or PtP polypeptide (including mutants, analogs, and functional equivalents), may be prepared by various molecular biology methods known in the art. Vectors comprising such sequences may be used as well.

## Methods of the invention

The subject matter described and claimed herein involves assays for measuring enzymatic activity. More particularly, the described subject matter relates to a rapid, sensitive, reliable and robust homogeneous assay for measuring meprin activity which comprises a coupled enzyme assay.

Generally, the methods may be used to measure meprin activity; identify modulators of meprin, identify competitive inhibitors of meprin, and identify allosteric modulators of meprin.

In a preferred embodiment, a method for identifying a compound that modulates, in particular inhibits meprin activity, is provided, said method comprising the steps of:

(a) providing a reaction mixture comprising a meprin and an auxiliary enzyme;
(b) adding a test compound to the reaction mixture;
(c) incubating the reaction mixture of step (b);
(d) adding a labeled substrate to the reaction mixture;
(e) incubating the reaction mixture of step (d);
(f) optionally terminating the reaction;
(g) measuring the amount of free label in the reaction mixture; and
(h) determining if the test compound inhibits meprin activity by comparing the amount of free label from the reaction mixture with the amount of free label from a control reaction mixture to which no test compound was added, wherein a reduced amount of free label in the reaction mixture compared to the amount of free label in the control reaction mixture is indicative of inhibition of the enzymatic catalytic activity of a meprin by the test compound

There are many experimental conditions that may be used in the methods described and claimed herein. For example, in some embodiments, the label used is a radioactive, fluorogenic, chromogenic or enzymatic label. Suitable radioactive labels are for example $^{14}C$ or $^{3}H$. Preferably, peptide substrates are used in the methods of the invention, wherein said peptides are labeled with a fluorogenic or chromogenic label. More preferably, the label used in the methods of the invention is a chromogenic label. Most preferably, the chromogenic label used in the methods of the invention is selected from 7-amino-4-methylcoumarin (AMC) and p-nitroaniline (pNA).

The peptide, which is labeled and used as labeled substrate in the methods of the invention is suitably an oligopeptide, preferably an oligopeptide consisting of 4 to 12 amino acids plus label, more preferably 5 to 11 amino acids plus label, or 6 to 10 amino acids plus label, most preferably 7 to 9 amino acids plus label. Further preferably, the last three amino acids at the C-terminus of the oligopeptide are represented by the amino acid sequence $X_{10}$-$X_{11}$-P. The chromogenic label is preferably attached at the C-terminus of this $X_{10}$-$X_{11}$-P sequence.

In a particularly preferred embodiment, the labeled substrate is a labeled oligopeptide of formula (I),

$$X_1X_2X_3X_4X_5X_6X_7X_SX_9X_{10}X_{11}P\text{-label} \qquad (I)$$

wherein

$X_1$ may be a peptide or any amino acid including proteinogenic amino acids, non-proteinogenic amino acids, L-amino acids and D-amino acids;

$X_2$, $X_3$, $X_4$, $X_5$, $X_6$, $X_7$, $X_8$ and $X_9$ may be any amino acid including proteinogenic amino acids, non-proteinogenic amino acids, L-amino acids and D-amino acids; and at least one of $X_1$, $X_2$, $X_3$, $X_4$, $X_5$, $X_6$, $X_7$, $X_8$ and $X_9$ is present and the others may be absent; and

the label is selected AMC and pNA.

In preferred embodiments of the invention

$X_1$ is present and $X_2$, $X_3$, $X_4$, $X_5$, $X_6$, $X_7$, $X_8$ and $X_9$ are absent, or

$X_1$, and $X_2$ are present and $X_3$, $X_4$, $X_5$, $X_6$, $X_7$, $X_8$ and $X_9$ are absent, or

$X_1$, $X_2$ and $X_3$ are present and $X_4$, $X_5$, $X_6$, $X_7$, $X_8$ and $X_9$ are absent, or

$X_1$, $X_2$, $X_3$ and $X_4$ are present and $X_5$, $X_6$, $X_7$, $X_8$ and $X_9$ are absent, or

$X_1$, $X_2$, $X_3$, $X_4$ and $X_5$ are present and $X_6$, $X_7$, $X_8$ and $X_9$ are absent, or

$X_1$, $X_2$, $X_3$, $X_4$, $X_5$ and $X_6$ are present and $X_7$, $X_8$ and $X_9$ are absent, or

$X_1$, $X_2$, $X_3$, $X_4$, $X_5$, $X_6$ and $X_7$ are present and $X_8$ and $X_9$ are absent, or

$X_1$, $X_2$, $X_3$, $X_4$, $X_5$, $X_6$, $X_7$ and $X_8$ are present and $X_8$ and $X_9$ are absent, or

all of $X_1$, $X_2$, $X_3$, $X_4$, $X_5$, $X_6$, $X_7$, $X_8$ and $X_9$ are present.

More preferably, $X_1$, $X_2$, $X_3$, $X_4$, $X_5$ and $X_6$ are present and $X_7$, $X_8$ and $X_9$ are absent.

In a further preferred embodiment, $X_1$, $X_2$, $X_3$, $X_4$, $X_5$, $X_6$, $X_7$, $X_8$ and $X_9$ are selected from the group consisting of

| Amino Acid | One-Letter Symbol | Three-Letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

In a more preferred embodiment, the labeled substrate used in the methods according to the invention is a labeled oligopeptide of formula (II)

$$X_4 X_5 X_6 X_7 X_S X_9 X_{10} X_{11} P\text{-label} \qquad (II)$$

wherein

$X_4$ is any amino acid or a peptide;

$X_5$ is any amino acid;

$X_6$ is any amino acid;

$X_7$ is amino acid selected from the group consisting of A, D, E, I, L, P, S, T, V, and Y;

$X_8$ is amino acid selected from the group consisting of A, D, E, I, L, P, Q, S, T, Q, and V;
$X_9$ is amino acid selected from the group consisting of A, D, E, L, N, Q, S;
$X_{10}$ is amino acid selected from the group consisting of D, E and T;
$X_{11}$ is amino acid selected from the group consisting of A, I, L, V, and F; and the label is pNA or AMC.

Preferably, $X_4$ is K.
Preferably, $X_5$ is K.
Preferably, $X_6$ is G.
Preferably, $X_7$ is Y.
Preferably, $X_8$ is V.
Preferably, $X_9$ is A.
Preferably, $X_{10}$ is D.
Preferably, $X_{11}$ is A.
Most preferably, $X_{10}X_{11}P$ is DAP.
Further preferably, $X_7$ is not W.
Further preferably, $X_8$ is not K or R.
Further preferably, $X_9$ is not I, K or R.
Further preferably, $X_{11}$ is not D, E, K, or L.
In a most preferred embodiment, the labeled substrate used in the methods according to the invention is a labeled oligopeptide of formula (III)

$$\text{KKGYVADAP-label} \qquad (\text{III}),$$

wherein the label is selected from AMC and pNA.

When the labeled substrate used in the methods according to the invention is a labeled oligopeptide of formula (II), said labeled substrate is in a particularly preferred embodiment KKGYVADAP-pNA or KKGYVADAP-AMC.

The reaction may optionally be terminated by addition of acid, base or by heat quench. The incubation period for the reaction may be for about 1 min to 1 hour, and the incubation temperature may be in a range of 25°C to 45°C. The reaction mixture may comprise about 0.01 - 4 μg of a meprin, such as meprin α or meprin β, and may further comprise about 0.05 - 2 unit auxiliary enzyme.

The amount of auxiliary enzyme can, e.g. be calculated according to the following equation:

$$V_2 = -K_{m2} \times \ln(1 - [\text{DAP-}p\text{NA}]_t / [\text{DAP-}p\text{NA}]_{ss})/t$$

$V_2$ is determined as the maximal velocity for the substrate cleavage and can be calculated from the determined Michaelis constant $K_{m2}$ (see Examples).

Meprins are desirable drug targets due to their role(s) in a variety of pathologic processes and related disease states. However, technical difficulties associated with assaying meprin activity have hindered progress in identifying meprin-related drugs. One of the technical challenges has been the lack of a convenient, robust, and economical enzyme assay that would facilitate high-throughput screening (HTS) for meprin inhibitors.

The conventional meprin assays are not practical for HTS. To fill this long-felt need, the inventors developed a homogenous coupled enzyme assay method using the labeled peptide substrates described above and an auxiliary enzyme. The assay involves monitoring a meprin reaction, in which the enzyme catalyzes the cleavage of N-terminal amino acids from the labeled peptide substrate to form labeled DAP (DAP-label). The auxiliary enzyme is preferably a prolyl tripeptidyl aminopeptidase (PtP), such as PtP from *Porphyromonas gingivalis*. PtP catalyzes the cleavage of the label from the labeled DAP. This assay is amenable to HTS and may be used to identify meprin modulators, in particular meprin inhibitors.

In preferred embodiments, the methods of the invention are thus employed in a high throughput screen format.

Further contemplated are kits useful for practicing the methods disclosed and claimed herein. For example, one embodiment may be a kit used for identifying one or more compounds that modulate meprin activity, comprising meprin α and/or meprin β, a labeled peptide substrate such as KKGYVADAP-pNA and/or KKGYVADAP-AMC, an auxiliary enzyme, such as PtP, and instructions for identifying one or more compounds that modulate meprin enzymatic activity.

The assays described herein offer many advantages over known methods for assaying meprin activity. First, the instant assay is not cumbersome as are known methods. The instant assay may be performed in a single reaction vessel and does not require any transfer of materials. The instant assays are simple, homogenous assays involving very few steps. The assay reagents are added and incubated and free label can be detected by continuous absorbance or fluorescence measurements. Optionally, the reaction may be terminated, e.g. by addition of acid, base or heat quench.

Readout can for example simply performed in microplate readers known in the art. In contrast, the conventional meprin assays are based on the cleavage of internally quenched substrates by meprin and show substrate inhibition. Here, the substrate inhibition prevents the assessment of inhibition at substrate concentrations of higher than Km. Therefore, the determination of inhibition constants is hampered, because both substrate and inhibitor present inhibit the meprin activity at the same time.

Additionally, the instant assays provide rapid results, making it suitable for use in a HTS setting. The known methods for assaying meprin activity are time- consuming and cannot be employed or adapted to perform adequately in a HTS operation.

Furthermore, the instant assays: 1) are relatively inexpensive to perform; 2) employ small amounts of enzymes that may be isolated as described herein, or may be acquired from commercial sources; and 3) are robust and reproducible.

Finally, the assay is adaptable. For example, partially purified (e.g., avidin-Sepharose affinity chromatography) meprin enzyme or PtP auxiliary enzyme may be used. Thus there is no need for highly purified enzyme in the instant assay which saves time and reduces expense.

## Detailed description of preferred embodiments of the invention

As described above, one embodiment described herein is a method for identifying a compound that modulates meprin activity. A meprin or a fragment thereof is added to the assay mixture. In preferred embodiments, the methods are conducted as a high throughput screen. For example, the reaction may be carried in a welled plate, such as a 96- or 384-welled plate.

The method comprises the steps of:

(a) providing a reaction mixture comprising a meprin and an auxiliary enzyme;
(b) adding a test compound;
(c) incubating the reaction mixture of step (b);
(d) adding a labeled substrate to the reaction mixture;
(e) incubating the reaction mixture of step (d);
(f) optionally terminating the reaction;
(g) measuring the amount of free label in the terminated reaction mixture; and
(h) determining if the test compound inhibits meprin activity by comparing the amount of free label from the terminated reaction mixture with the amount of free label from a control reaction mixture to which no test compound was added, wherein a reduced amount of free label in the terminated reaction mixture compared to the amount of free label in the control reaction mixture is indicative of inhibition of the enzymatic catalytic activity of a meprin by the test compound.

The first step "(a)" involves providing a reaction mixture comprising meprin and an auxiliary enzyme, such as PtP in an assay buffer. The reaction mixture comprises about 0.05 - 4 $\mu$g meprin, and preferably comprises about 0.5 $\mu$g of meprin. The enzyme used may be partially purified. The reaction mixture comprises about 0.05-1 units of PtP and preferably comprises about 0.5 units of PtP.

The second step "(b)" involves adding a test compound, which is capable of modulation meprin activity, to the reaction mixture of step "(a). The test compound is suitably added in a concentration in the range of $10^{-3}$ to $10^{-12}$ M, preferably in the range of $10^{-4}$ to $10^{-11}$ M or $10^{-5}$ to $10^{-10}$ M, more preferably in the range of $10^{-6}$ to $10^{-9}$ M, most preferably in the range of $10^{-7}$ to $10^{-8}$ M. Suitably, the test compound added in step "(b) does not affect the activity of the auxiliary enzyme.

The third step "(c)" involves incubation the reaction mixture of step "(b)". This is a preincubation step to allow the test compound to react and/or interact with meprin. Incubation in step "(c)" is performed for several minutes to several hours, preferably for several minutes, such as for a duration in range between 5 minutes to 30 minutes, preferably 5 minutes to 20 minutes, more preferably 5 minutes to 20 minutes, most preferably 5 minutes to 15 minutes, such as 10 minutes.

The fourth step "(d)" involves adding a labeled substrate to the reaction mixture. In a preferred embodiment, the labeled substrate is labeled at the C-terminus. The labeled substrate may be labeled with any agent that allows for subsequent detection of the free label. Exemplary labels are radioactive, fluorogenic, chromogenic or enzymatic labels. It is within the skill of an ordinary artisan to determine an appropriate label and how to make the labeled substrate containing the selected label. In a preferred embodiment, the label is a chromogenic label selected from the group consisting of pNA and AMC. The labeled substrate is more preferably a compound of formula (I), most preferably a compound of formula (II), particularly preferred a compound selected from KKGYVADAP-pNA or KKGYVADAP-AMC. The labeled substrate is suitably added at a concentration in the range between $10^{-2}$ to $10^{-12}$ M, preferably in the range

of $10^{-3}$ to $10^{-10}$ M, more preferably in the range of $10^{-4}$ to $10^{-8}$ M, most preferably in the range of $10^{-5}$ to $10^{-6}$ M.

The fifth step "(e)" involves incubation the reaction mixture of step "(d)". The incubation step allows for the reagents to react and to generate enzymatic products, such as the free labels, which are released from the labeled substrates. Incubation is performed for several minutes to several hours, preferably for several minutes, such as for 5 minutes, 10 minutes, 15 minutes, 20 minutes, 25 minutes or 30 minutes, more preferably for 15 minutes, 20 minutes, 25 minutes, most preferably for 20 minutes.

Preincubation of step "(c)" and incubation of step "(e)" may be performed at the same temperature or at different temperatures. Preferably, preincubation and incubation of the reaction mixture are performed at the same temperature. More preferably, said incubation and/or said preincubation is performed at a temperature in the range of 25°C to 45°C, preferably in the range of 27°C to 37°C, more preferably in the range of 30°C to 35°C, most preferably at 30°C.

Determining the amount of free label, assay buffer and pH-value used in the reaction mixture is a matter within the skill of an ordinary artisan. Exemplary assay buffers comprises about 20 mM Tris-HCl, pH 7.5, or 20 mM HEPES, 10 mM $CaCl_2$, 0.05% Brij, pH 7.4.

The optional sixth step ("f") involves termination of the reaction. Exemplary methods for terminating the reaction include, but are not limited to, addition of acid or base to the reaction mixture, and heat quench.

In a preferred embodiment, the methods of the invention do not contain step "(f)", i.e. the methods of the invention do not contain a step of termination of the reaction. More preferably, the methods of the invention are performed as continuous assays, where the formation of free label is monitored over a suitable time period in a suitable means for detecting and/or measuring the free label. Accordingly, the seventh step ("g") involves measuring the amount of free labeled in the (optionally terminated) reaction mixtures. Any procedure for measuring the amount of a labeled molecule may be used. Exemplary detection methods, include, but are not limited to, scintillation counting, and scintillation proximity, spectroscopy. Preferably, in accordance with the labels used in the methods of the invention, spectrophotometric and/or fluorometric methods are used for detecting the free label in the reaction mixture. In a further preferred embodiment, step "(f)" is absent and the measurement of the release of the free label occurs already during the incubation in step "(d)", i.e. steps "(e)" and "(g)" are combined to perform a continuous monitoring of the release of the free label.

The final step "(h)" involves comparing the amount of free label detected in the (optionally terminated) reaction mixture with the amount of free label in a control reaction mixture to which no test compound was added. The control reaction mixture is prepared following the steps described above, except no test compound is added. If the amount of free label in the optionally terminated reaction mixture is more or less than that in the control reaction mixture, this suggests that the test compound modulates meprin activity. If the amount of free label in the optionally terminated reaction mixture is less than that in the control reaction mixture, this suggests that the test compound inhibits meprin activity, which is particularly desired.

**Additional methods**

As mentioned before, two forms of meprin, meprin $\alpha$ and meprin $\beta$ exist. The term "meprin" as used herein thus includes both forms, meprin $\alpha$ and meprin $\beta$. In a preferred embodiment of the invention, the meprin used in the methods of the invention is meprin $\alpha$. In another preferred embodiment, the meprin used in the methods of the invention is meprin $\beta$. In a more preferred embodiment, the invention thus provides methods for identifying inhibitors that are specifically inhibiting either meprin $\alpha$ or meprin $\beta$.

In a particularly preferred embodiment, the invention provides a method for identifying a compound that specifically inhibits meprin $\alpha$ activity, comprising the steps of:

(a) providing a reaction mixture comprising meprin $\alpha$ and an auxiliary enzyme;
(b) adding a test compound and incubating the reaction mixture;
(c) incubating the reaction mixture of step (c);
(d) adding a labeled substrate to the reaction mixture;
(e) incubating the reaction mixture of step (d);
(f) optionally terminating the reaction;
(g) measuring the amount of free label in the reaction mixture; and
(h) determining if the test compound specifically inhibits meprin $\alpha$ activity by comparing the amount of free label from the reaction mixture with the amount of free label from a control reaction mixture which comprises meprin $\beta$, wherein a reduced amount of free label in the reaction mixture compared to the amount of free label in the control reaction mixture is indicative of the specific inhibition of the enzymatic catalytic activity of meprin $\alpha$ by the test compound.

Steps (a) through (g) are essentially the same steps as steps (a) through (g) described above with respect to the methods for identifying meprin modulators. However, the last step "(h)" is different from above. Step "(h)" comprises determining if the test compound specifically inhibits meprin $\alpha$ activity by comparing the amount of free label from the reaction mixture with the amount of free label from a control reaction mixture which comprises meprin $\beta$. A reduced amount of free label in the reaction mixture compared to the amount of free label in the control reaction mixture is indicative of the specific inhibition of the enzymatic catalytic activity of meprin $\alpha$ by the test compound, but less or no inhibition of meprin $\beta$ by the test compound.

In a further most preferred embodiment, the invention provides method for identifying a compound that specifically inhibits meprin $\beta$ activity, comprising the steps of:

(a) providing a reaction mixture comprising meprin $\beta$ and an auxiliary enzyme;
(b) adding a test compound and incubating the reaction mixture;
(c) incubating the reaction mixture of step (c);
(d) adding a labeled substrate to the reaction mixture;
(e) incubating the reaction mixture of step (d);
(f) optionally terminating the reaction;
(g) measuring the amount of free label in the reaction mixture; and
(h) determining if the test compound specifically inhibits meprin $\beta$ activity by comparing the amount of free label from the reaction mixture with the amount of free label from a control reaction mixture which comprises meprin $\alpha$, wherein a reduced amount of free label in the reaction mixture compared to the amount of free label in the control reaction mixture is indicative of the specific inhibition of the enzymatic catalytic activity of meprin $\beta$ by the test compound.

Steps (a) through (g) are essentially the same steps as steps (a) through (g) described above with respect to the methods for identifying meprin modulators. However, the last step "(h)" is different from above. Step "(h)" comprises determining if the test compound specifically inhibits meprin $\beta$ activity by comparing the amount of free label from the reaction mixture with the amount of free label from a control reaction mixture which comprises meprin $\alpha$. A reduced amount of free label in the reaction mixture compared to the amount of free label in the control reaction mixture is indicative of the specific inhibition of the enzymatic catalytic activity of meprin $\beta$ by the test compound, but less or no inhibition of meprin $\alpha$ by the test compound.

The methods described in the section above (i.e., Methods for identifying a compound that modulates meprin activity) can be readily adapted to assay other enzymatic characteristics. For example, the methods of section III can be modified to produce methods for: measuring the potency (e.g., IC50) of a test compound; differentiating a competitive inhibitor from a non-competitive inhibitor; and for assaying enzymatic catalytic activity of meprin. Embodiments of such methods are described below.

**Method for measuring the IC50 value of a test compound**

One embodiment is a method for measuring the IC50 value of a test compound against meprin. As is well known, IC50 is the concentration of a compound that yields 50% inhibition of enzymatic activity. The method comprises the steps of

(a) providing a reaction mixture comprising a meprin and an auxiliary enzyme;
(b) adding increasing amounts of a test compound;
(c) incubating the reaction mixtures of step (b);
(d) adding a labeled substrate to the reaction mixtures;
(e) incubating the reaction mixtures of step (d);
(f) optionally terminating the reaction;
(g) measuring the amount of free label in the terminated reaction mixture; and
(h) determining the IC50 value of the test compound upon the amount of free label in each reaction mixture.

Steps (a) through (g) are essentially the same steps as steps (a) through (g) described above with respect to the methods for identifying meprin modulators. However, the last step "(h)" is different from above. Step "(h)" comprises determining the IC50 value of the test compound against meprin based upon the amount of free label in each reaction mixture. The amount of test compound that may be used is any amount that enables a determination of the IC50 value. Determining the amount of test compound for use in the method is within the level of skill of an ordinary artisan. For example, the activity in the presence of 8-11 half-log concentrations of the compound may be determined. The control activity in the absence of the compound (subtracted from no enzyme blank) serves as 100% activity and the percent inhibition at each compound concentration is calculated [1- activity with compound/control activity] x 100. The percent inhibition is plotted against the log of the compound concentration and the data fitted to the following equation $Y = A +$

((B-(A)/((1+((C/x)^(D)))). The above-described method can be adapted to assay the IC50 of test compounds for other enzymes as well.

**Method for differentiating a competitive inhibitor from a non-competitive inhibitor**

Another embodiment is a method for differentiating a competitive inhibitor of meprin from a non-competitive inhibitor of meprin. The method comprises the steps of:

(a) providing a reaction mixture comprising a meprin and an auxiliary enzyme;
(b) adding a meprin inhibitor;
(c) incubating the reaction mixture of step (b);
(d) adding a labeled substrate to the reaction mixture;
(e) incubating the reaction mixture of step (d);
(f) optionally terminating the reaction;
(g) measuring the amount of free label in the terminated reaction mixture; and
(h) determining if the meprin inhibitor is a competitive inhibitor of a meprin or a non-competitive inhibitor of a meprin based upon the amount of free label in the reaction mixture.

Steps (a) through (g) are essentially the same steps as steps (a) through (g) described above with respect to the methods for identifying meprin modulators. A known meprin inhibitor is used in the assay rather than a "test compound", and the last step "(h)" is different. The test meprin inhibitor is preferably a compound known to inhibit enzymatic activity and is present in the reaction mixture at about 0.015-30 $\mu$M. Step (h) comprises determining if the test inhibitor is a competitive or non-competitive inhibitor of meprin, based upon the amount of free label, such as pNA or AMC, present in the reaction mixture. For example, a known competitive inhibitor of meprin is actinonin, and as illustrated herein, inhibited meprin $\alpha$ with a $K_i$ value of $3.2 \times 10^{-9} \pm 8.6 \times 10^{-10}$ M and meprin $\beta$ with a $K_i$ value of $2.7 \times 10^{-7} \pm 7.1 \times 10^{-9}$ M. One skilled in the art would be able to evaluate data obtained from known competitive or non-competitive inhibitors and compare that data with putative inhibitors and determine whether the putative inhibitor is a competitive or non-competitive inhibitor.

**Method for assaying meprin catalytic activity**

Another embodiment is a method for assaying meprin catalytic activity. The method comprises the steps of :

(a) providing a reaction mixture comprising a meprin and an auxiliary enzyme;
(b) adding a labeled substrate to the reaction mixture;
(c) incubating the reaction mixture;
(d) optionally terminating the reaction; and
(e) measuring the amount of free label in the terminated reaction mixture, wherein the presence of free label is indicative of enzymatic catalytic activity of a meprin.

Steps (a) through (e) are essentially the same steps as steps (a), and (d) through (f) described above with respect to the methods for identifying meprin modulators. However, the reaction mixture comprises a sample known to or believed to comprise a meprin and step (e) is different from step (g) of the method for identifying meprin modulators.
Step (e) comprises measuring the amount of free label in the optionally terminated reaction mixture, wherein the presence of free labeled, such as pNA or AMC, is indicative of enzymatic catalytic activity of meprin. When the sample is known to contain a meprin, the assay may be performed to measure the amount and/or activity of the enzyme present in the sample.
When it is not known whether a sample contains meprin, the assay may be performed to identify the presence and/or activity of meprin in that sample.
Such a sample could be the product of a meprin purification procedure, for example.

**Kits**

Other embodiments disclosed and claimed herein are kits that facilitate the use of the methods described herein. For example, the kits may be used to perform methods for: identifying a compound that modulates meprin activity; measuring the IC50 and/or $K_i$ value value of a test compound; differentiating a competitive inhibitor of meprin from a non-competitive inhibitor of meprin; and for assaying meprin catalytic activity.
The kit comprises meprin, an auxiliary enzyme such as PtP, a labeled substrate such as an oligopeptide C-terminally labeled with pNA or AMC, and instructions for performing the methods described herein.

In some embodiments, a container is provided for carrying out the reaction. The contents of the kit may be provided in concentrated solutions or in ready-to-use aliquots.

## Examples

The following Examples have been included to illustrate preferred embodiments of the methods/assays disclosed and claimed herein. The following Examples are illustrative and changes, modifications or alterations may be employed by those of ordinary skill in the art without departing from the spirit and scope of the subjected matter disclosed and claimed herein.

In the following examples, *p*-nitroaniline and molecular mass standards for SDS-PAGE were provided by Sigma (Deisenhofen, Germany); His-Trap, Butyl Sepharose and HiPrep columns were obtained from GE Healthcare Life Science (Piscataway, USA).

## Example 1: Synthesis of H-Lys-Lys-Gly-Tyr-Val-Ala-Asp-Ala-Pro-pNA

The peptide was synthesized according to a modified procedure as described by Abbenante *et al*. (Abbenante, G., Leung, D., Bond, T., Fairlie, D. P., Lett Pept Sci. 2000, 7(6), 347-351, 10.1007/BF02443598).

Step 1: Boc-Lys(Boc)-Lys(Boc)-Gly-Tyr(tBu)-Val-Ala-Asp(tBu)-Ala-Pro-p-aminoanilide

2-Chlorotriltyl resin (410 mg, 0.5 mmol, 1 eq) was added to a solution of DIPEA (1.71 ml, 10 mmol, 20 eq) and p-Phenylendiamine hydrochloride (578 mg, 4 mmol, 8 eq) in DMF (10 ml). The resin was shaken at room temperature for 2 days, washed with DMF and subsequently shaken with a mixture of DMF/MeOH (9:1, 20 ml). After washing with MeOH and DCM the resin was directly used for Fmoc-SPPS.

The peptide was synthesized according to standard Fmoc/tBu synthesis. Briefly, the amino acids were coupled using TBTU and NMM in DMF at room temperature for 30 minutes (ratio resin : amino acid : TBTU : NMM = 1:4:4:8). All amino acid couplings were performed as double couplings. Fmoc-deprotection was carried out using 20% piperidine in DMF for 4 minutes + 8 minutes.

The fully protected peptide (Boc-Lys(Boc)-Lys(Boc)-Gly-Tyr(tBu)-Val-Ala-Asp(tBu)-Ala-Pro-p-aminoanilide was cleaved from the resin by means of 1% TFA/DCM at room temperature for 2x2 hour. The combined filtrates were evaporated and used without further purification.

Step 2: H-Lys-Lys-Gly-Tyr-Val-Ala-Asp-Ala-Pro-pNA

The residue was redissolved in water/acetonitrile (1:1, 50 ml) and treated with Oxone® (922 mg, 3 mmol, 6 eq). The mixture was stirred over night at room temperature. The oxidized peptide-pNA was extracted by means of EtOAc (3x25 ml). The combined organic layers were dried over $Na_2SO_4$ and evaporated. Deprotection of the sidechains was achieved by treating the crude peptide with TFA/DCM (1:1, 20 ml) and TIS (204 µl, 1 mmol) for 2 hour at room temperature. The volatiles were evaporated and the residue was purified by semi-preparative HPLC.

## Example 2: Synthesis of H-Lys-Lys-Gly-Tyr-Val-Ala-Asp-Ala-Pro-AMC

Step 1: Boc-Lys(Boc)-Lys(Boc)-Gly-Tyr(tBu)-Val-Ala-Asp(tBu)-Ala-OH

The peptide was synthesized using Fmoc-Ala-2CT resin (207 mg, 0.12 mmol). After deprotection (20% piperidine/DMF; 4 minutes + 8 minutes) the peptide was synthesized according to standard Fmoc/tBu synthesis. Briefly, the amino acids were coupled using TBTU and NMM in DMF at room temperature for 30 minutes (ratio resin : amino acid : TBTU : NMM = 1:4:4:8). All amino acid couplings were performed as double couplings. Fmoc-deprotection was carried out using 20% piperidine in DMF for 4 minutes + 8 minutes. The fully protected peptide was cleaved from the resin by means of TFE/DCM (2:8 (v/v), 10 ml, 2 x 2 hours). The combined filtrates were evaporated and the residue was used without further purification.

Step 2: H-Lys-Lys-Gly-Tyr-Val-Ala-Asp-Ala-Pro-AMC

Boc-Lys(Boc)-Lys(Boc)-Gly-Tyr(tBu)-Val-Ala-Asp(tBu)-Ala-OH (135 mg, 0.107 mmol, 1 eq) was dissolved in DMF (6 ml) and treated with TBTU (34 mg, 0.107 mmol, 1 eq), NMM (24 µl, 0.214 mmol, 2 eq) and Pro-AMC*HCl (33 mg, 0.107 mmol, 1 eq). The mixture was stirred at room temperature for 2 hours. After evaporation of the volatiles, deprotection of the sidechains was achieved by treating the crude peptide with TFA/DCM (1:1, 20 ml) and TIS (204 μl, 1 mmol) for 2 hour at room temperature. The volatiles were evaporated and the residue was purified by semi-preparative HPLC.

*Synthesis of H-Asp-Ala-Pro-pNA*

Step 1: H-Ala-Pro-pNA*HCl

Boc-Ala-OH (547 mg, 2.9 mmol, 1 eq) was dissolved in THF (20 ml). CAIBE (374 μl, 2.9 mmol, 1 eq) and NMM (319 μl, 2.9 mmol, 1 eq) were added at 0°C. After stirring at 0°C for 20 minutes, H-Pro-pNA*HCl (780 mg, 2.9 mmol, 1 eq) and NMM (319 μl, 2.9 mmol, 1 eq) were added and stirring was continued for 1 hour. The temperature was raised to room temperature and stirring was continued over night. The volatiles were evaporated and the residue was dissolved in EtOAc (50 ml). The organic layer was washed with brine (3x25 ml) and sat. aqueous NaHCO$_3$ (3x25 ml), dried over Na$_2$SO$_4$ and evaporated. The residue was treated with HCl/Dioxane (20 ml) at room temperature. After stirring for 2 hours, the volatiles were evaporated. The residue was recrystallized from Et$_2$O/MeOH and used without further purification.

Step 2: H-Asp-Ala-Pro-pNA*HCl

Boc-Asp(OtBu)-OH (406 mg, 1.4 mmol, 1 eq) was dissolved in THF (20 ml). CAIBE (183 μl, 1.4 mmol, 1 eq) and NMM (154 μl, 1.4 mmol, 1 eq) were added at O°C. After stirring at 0°C for 20 minutes, H-Ala-Pro-pNA*HCl (481 mg, 1.4 mmol, 1 eq) and NMM (154 μl, 1.4 mmol, 1 eq) were added and stirring was continued for 1 hour. The temperature was raised to room temperature and stirring was continued over night. The volatiles were evaporated and the residue was dissolved in EtOAc (50 ml). The organic layer was washed with brine (3x25 ml) and sat. aqueous NaHCO$_3$ (3x25 ml), dried over Na$_2$SO$_4$ and evaporated. The residue was treated with HCl/Dioxane (20 ml) at room temperature. After stirring for 2 hours, the volatiles were evaporated. The residue was recrystallized from Et$_2$O/MeOH and purified by semi-preparative HPLC.

**Example 3: Cloning, expression and purification of h-Meprin β in *P. pastoris***

Cloning, expression and purification of h-meprin β was performed as described previously (Schlenzig, D., Wermann, M., Ramsbeck, D., Moenke-Wedler, T., Schilling, S., Protein expression and purification. 2015, 116, 75-81, 10.1016/j.pep.2015.08.001). In brief, *Pichia pastoris* strain X33 (Invitrogen) was transformed with pPICZαC containing commercially available human cDNA of h-meprin β (OriGene) applying standard cloning procedures (Sambrock, J., Fritsch, E. F., Maniatis, Cold Spring Harbor Laboratory Press, New York 1989). Expression of h-meprin β was carried out in a fermenter (Biostad B, B. Braun biotech) and the supernatant was harvested after 88 hour.
The N-terminally His$_6$-tagged h-meprin β was purified by immobilized metal ion affinity chromatography. The resulting pro-h-meprin β was activated by trypsin digestion. H-meprin β was purified with hydrophobic interaction chromatography and gel filtration. Finally, the protein was concentrated, desalted and kept at -80°C in 40 mM Tris, 100 mM NaCl; pH 7.6 until use.

**Example 4: Cloning, expression and purification of h-Meprin α in *Drosophila melanogaster* S2 cells**

Commercially available cDNA of human h-meprin α was cloned into pMT/BiP/V5-His C vector applying standard cloning procedures (Sambrock, J., Fritsch, E. F., Maniatis, Cold Spring Harbor Laboratory Press, New York 1989). Expression of h-meprin α, possessing an N-terminal His-tag was performed in *Drosophila melanogaster* S2 cells, which were stably transfected with pMT/BiP/V5-C-hMep α. Protein expression was induced after addition of 700 μM copper sulfate. After 48 hours, the culture media was harvested. The media was adjusted to 1.5 M ammonium sulfate and directly centrifuged (30,000 g; 30 minutes; 4°C) and purified using hydrophobic interaction expanded bed adsorption (HIC-EBA) chromatography. Afterward the column was washed with two column volumes washing buffer (30 mM Tris, 1.5 M ammonium sulfate; pH 7.5) and elution of the protein was achieved with elution buffer (30 mM Tris; pH 7.5) in a single step. The elution fraction was further purified utilizing size exclusion and anion exchange chromatography.

**Example 5: Cloning of Prolyl Tripeptidyl Aminopeptidase (PtP)**

The pQE30 vector containing the cDNA of ptpA *(pQE30-ptpA)* flanked by a Bam*HI and* Hind*III* restriction site was purchased from Centic Biotec (Heidelberg, Germany). To express a cytoplasmic protein, the sequence is lacking the corresponding amino acids 1-38 for the N-terminal membrane anchor (Banbula, A., Mak, P., Bugno, M., Silberring, J., Dubin, A., Nelson, D., Travis, J., Potempa, J., The Journal of biological chemistry. 1999, 274 (14), 9246-9252). For protein purification, a $His_6$-tag was added N-terminally of the ptpA.

*Expression and Purification of Prolyl Tripeptidyl Aminopeptidase in E. coli*

*The* pQE30-ptpA *plasmid was* transformed into *Escherichia coli* M15 and cultured in LB-broth containing 100 $\mu$g/ml ampicillin and 25 $\mu$g/ml kanamycin at 30°C for 18 h in a flask. The cells were suspended in 50 m*M* Tris-HCl buffer; pH 7.4, supplemented with 1 mg/ml lysozyme and were disrupted by sonication (Bandelin Sonopuls, Berlin, Germany) on ice using 9 cycles with a 10 s burst. The cell lysate was centrifuged at 50.000 *g* for 10 minutes and the supernatant was purified by a HIC column (His-Trap Crude, GE). The solution was loaded onto a Butyl Sepharose column for hydrophobic interaction chromatography and eluted with 30 m*M* Tris-HCl, 100 mM NaCl, 1.6 M ammonium sulfate buffer; pH 7.6. To avoid protein degradation, all steps were performed at 10°C.
The purified enzyme was desalted using a HiPrep 26/10 desalting column and eluted with 20 mM Tris-HCl, 100 mM NaCl buffer; pH 7.0, and concentrated to 2.85 mg/ml using a Vivaspin 20, 30.000 MWO (Satorius, Göttingen, Germany). To keep a robust enzyme activity over several months, the protein was supplemented with 2 mM DTE, shock frozen in liquid nitrogen and stored at -80°C. For analysis of protein concentration and purification, Bradford measurements, SDS-PAGE and Western-Blotting were executed (Bradford, M. M., Analytical biochemistry. 1976, 72, 248-254).

**Example 6: Meprin Assays**

*Spectrophotometric Assays*

The enzyme assay consisted of prolyl tripeptidyl aminopeptidase from *Porphyromonas gingivalis,* an appropriate amount of human h-meprin $\beta$ or $\alpha$ and the chromogenic substrate KKGYVADAP-pNA (stock solution in assay buffer) in a final volume of 0.25 ml h-meprin $\beta$ assay buffer (20 mM Tris-HCl; pH 7.5) or h-meprin $\alpha$ assay buffer (20 mM HEPES, 10 mM $CaCl_2$, 0,05% Brij; pH 7.4). After 10 minutes preincubation, the reaction was started by addition of the substrate. Subsequently, absorption was measured at 405 nm for 20 minutes with the Tecan Genios Pro Reader (Tecan) at 30°C. PtP activity was determined using an absorption coefficient of 5717 mol/l, which was obtained from a standard curve of *p*-nitroaniline under assay conditions. A molecular mass of 79500 Da was used to calculate the $K_{cat}$ value.
Prolyl tripeptidyl aminopeptidase activity was measured utilizing the substrate DAP-*p*NA. In order to maintain conditions identical to those used in the h-meprin $\beta$ assay, samples (250 $\mu$l) contained a final concentration of 0.2% (v/v) dimethyl sulfoxide. One unit is defined as the amount of enzyme that hydrolyses 1 $\mu$mol DAP-pNA per minute under the conditions described. The specific activity of the enzyme preparation was 7 units/mg.

*Fluorometric Assay*

Conversion of the fluorogenic substrate KKGYVADAP-AMC by PtP and h-meprin $\beta$ was analyzed according to assay conditions as described for the chromogenic substrate. Fluorescence was measured at excitation/emission wavelengths of 380/460 nm. The fluorescence coefficient of $8.5 \times 10^8$ mol/l for DAP-AMC was calculated from a standard curve of 7-Amino-4-methylcoumarin under assay conditions.

RESULTS AND DISCUSSION

Assay *Development*

Until now, kinetic parameters as inhibitor constants of meprins are obtained from hydrolysis of internally quenched substrates. Since this substrate reveals inhibitory effects on meprin, we hereby present an irreversible coupled continuous assay based on a chromogenic substrate and the auxiliary enzyme PtP. High expression level of PtP, meprin $\alpha$ and meprin $\beta$ were achieved in *E. coli, Drosophila melanogaster* S2 cells and in *P. pastoris,* respectively. Purification of the proteins resulted in adequate protein yields with virtually no impurities (Figure 1).
In the first reaction of the substrate conversion, KKGYVADAP-pNA is cleaved to DAP-*p*NA by meprin $\alpha$ or $\beta$. The second reaction, the release of *p*-nitroaniline by PtP can be measured in this assay and correlates to the amount of released DAP-*p*NA by meprin. Based on these terms, a rate equation for irreversible coupled enzyme assays was first described by McClure, giving the opportunity of calculating the required amount of an auxiliary enzyme to minimize the time to reach steady-state conditions (McClure, W. R., Biochemistry. 1969, 8 (7), 2782-2786; Schilling, S., Hoffmann, T., Wermann, M., Heiser, U., Wasternack, C., Demuth, H.-U., Analytical biochemistry. 2002, 303 (1), 49-56, 10.1006/abio.2001.5560). The amount of auxiliary enzyme was calculated as follows (Eq. 1):

$$V_2 = -K_{m2} \times \ln(1 - [DAP\text{-}p\mathrm{NA}]_t/[DAP\text{-}p\mathrm{NA}]_{ss})/t. \tag{1}$$

$V_2$ is determined as the maximal velocity for the substrate cleavage and can be calculated from the determined Michaelis constant $K_{m2}$. For the conversion of DAP-$p$NA by PtP, a $K_{m2}$ value of 501 $\mu$M $\pm$ 20 $\mu$M was obtained. The substrate concentration is termed by [DAP-$p$NA]$_t$ at time $t$ and [DAP-$p$NA]$_{ss}$ under steady-state conditions. Equation (1) requires stable initial substrate concentrations at time $t$, i.e. the first reaction is irreversible and the second reaction follows a first-order rate law. The conversion of KKGYVADAP-$p$NA was compared to the fluorogenic substrate KKGYVADAP-AMC. Thus, parameters obtained for DAP-$p$NA were also estimated for DAP-AMC. We determined a $K_{m2}$ value of 123 $\mu$M $\pm$ 15 $\mu$M. Assuming that one unit of auxiliary enzyme is defined as the amount of PtP that hydrolyses 1 $\mu$mol DAP-$p$NA per minute, the calculated amount of PtP from equation (1) to reach a steady-state concentration of 95% for DAP-$p$NA and DAP-AMC was 2.2 U and 1.1 U, respectively under the conditions described before. According to the calculations for irreversible coupled continuous assays, linear progression curves were obtained after adjustment of assay conditions (Figure 2). No increase in fluorescence (Figure 2, A) or absorbance (Figure 2, B) could be monitored in the absence of PtP or meprin $\beta$, demonstrating the requirement of both enzymes to cleave the substrate.

*Assay Application*

Considering the previous explained calculations, kinetic parameters for the conversion of the chromogenic substrate KKGYVADAP-$p$NA and the fluorogenic substrate KKGYVADAP-AMC by meprin $\beta$ were examined. Whereas, OCK+ (Abz-MGWMDEIDK(Dnp)SG-OH) showed substrate inhibition at concentrations above 40 $\mu$M and S2 (Abz-YVAEAPK(Dpn)G-OH) at concentrations above 100 $\mu$M, no inhibitory effect could be observed with KKGYVADAP-$p$NA and KKGYVADAP-AMC at concentrations up to 2 mM (Figure 3) (Bertenshaw, G. P., Villa, J. P., Hengst, J. A., Bond, J. S., Biological chemistry. 2002, 383 (7-8), 1175-1183, 10.1515/BC.2002.129; Schlenzig, D., Wermann, M., Ramsbeck, D., Moenke-Wedler, T., Schilling, S., Protein expression and purification. 2015, 116, 75-81, 10.1016/j.pep.2015.08.001). A Michaelis constant of $1.8 \times 10^{-4} \pm 3.2 \times 10^{-5}$ M and $7.4 \times 10^{-5} \pm 1.1 \times 10^{-5}$ M was determined for KKGYVADAP-pNA and KKGYVADAP-AMC, respectively (Table 1). The $K_{cat}$ value of 20 $\pm$ 4.1 s$^{-1}$ was calculated for KKGYVADAP-$p$NA using a molecular mass of a monomeric meprin $\beta$ protein of 67 kDa. This result corresponds to the catalytic constant estimated from the fluorogenic substrate.

Since fluorogenic substrates frequently intercalate with inhibitors, only KKGYVADAP-pNA was used to determine inhibitory constants ($K_i$). Estimated $K_i$ values of the known meprin $\alpha$ and $\beta$ inhibitor actinonin were taken from substrate conversion by meprins at six inhibitor and four substrate concentrations (Figure 4, A and B, Table 2). Inhibitory constants are $3.2 \times 10^{-9} \pm 8.6 \times 10^{-10}$ M for meprin $\alpha$ and $2.7 \times 10^{-7} \pm 7.1 \times 10^{-9}$ M for meprin $\beta$ and correspond to previous published data utilizing non-coupled assays with internally quenched substrates (Table 2) (Schlenzig, D., Wermann, M., Ramsbeck, D., Moenke-Wedler, T., Schilling, S., Protein expression and purification. 2015, 116, 75-81, 10.1016/j.pep.2015.08.001; Kruse, M.-N., Becker, C., Lottaz, D., Kohler, D., Yiallouros, I., Krell, H.-W., Sterchi, E. E., Stocker, W., The Biochemical journal. 2004, 378 (Pt 2), 383-389, 10.1042/BJ20031163; Broder, C., Becker-Pauly, C., The Biochemical journal. 2013, 450 (2), 253-264, 10.1042/BJ20121751). The inhibition of meprin $\alpha$ and $\beta$ appears to be competitive, since all four lines displayed in a Dixon plot intersect above the x-axis lines (Figure 4, C and D).

**Table 1** Kinetic parameters of meprin $\beta$ for hydrolysis of the different substrates. Values are displayed as means $\pm$ S.D. + (Schlenzig, D., Wermann, M., Ramsbeck, D., Moenke-Wedler, T., Schilling, S., Protein expression and purification. 2015, 116, 75-81, 10.1016/j.pep.2015.08.001)

| | $K_M$ (M) | $K_{cat}$ (s$^{-1}$) | $K_{cat}/K_M$ (s$^{-1}$M$^{-1}$) |
|---|---|---|---|
| Ac-RGRGYVADAP-$p$NA | $14.0 \times 10^{-5} \pm 7.7 \times 10^{-5}$ | $19.9 \pm 10.2$ | $15.0 \times 10^4 \pm 1.1 \times 10^5$ |
| Ac-KKGYVADAP-$p$NA | $36.0 \times 10^{-5} \pm 1.6 \times 10^{-4}$ | $20.0 \pm 3.5$ | $6.4 \times 10^4 \pm 2.5 \times 10^4$ |
| KKGYVADAP-$p$NA | $18.0 \text{v} 10^{-5} \pm 3.2 \times 10^{-5}$ | $20.0 \pm 4.1$ | $11.0 \times 10^4 \pm 4.8 \times 10^3$ |
| Ac-KKGYVADAP-AMC | $6.4 \times 10^{-5} \pm 1.6 \times 10^{-5}$ | $6.3 \pm 1$ | $10.0 \times 10^4 \pm 1.4 \times 10^4$ |
| KKGYV ADAP-AMC | $7.4 \times 10^{-5} \pm 1.1 \times 10^{-5}$ | $4.3 \pm 6.1 \times 10^{-1}$ | $6.0 \times 10^4 \pm 1.6 \times 10^4$ |
| Abz-YVAEAPK(Dpn)G-OH+ | $2.7 \times 10^{-5} \pm 1.3 \times 10^{-6}$ | $35.0 \pm 2.5$ | $129.0 \times 10^4 \pm 3.0 \times 10^4$ |

**Table 2** Half maximal inhibitory concentrations (IC$_{50}$) for actinonin, inhibiting meprin $\alpha$ and meprin $\beta$. Fluorogenic substrates are Abz-YVAEAPK(Dpn)G-OH and Abz-YVADAPK(Dpn)G-OH. For the coupled continuous assay, KKGYVADAP-*p*NA was used as chromogenic substrate. Values are displayed as means $\pm$ S.D.; + (Schlenzig, D., Wermann, M., Ramsbeck, D., Moenke-Wedler, T., Schilling, S., Protein expression and purification. 2015, 116, 75-81, 10.1016/j.pep.2015.08.001), ‡ unpublished data,

| | IC50 (M) for h-meprin $\alpha$ | IC50 (M) for h-meprin $\beta$ |
|---|---|---|
| KKGYV ADAP-*p*NA | $1.2\times10^{-8} \pm 4.0\times10^{-10}$ | $7.6\times10^{-7} \pm 2.6\times10^{-7}$ |
| Abz-YVAEAPK(Dpn)G-OH+ | | $4.4\times10^{-7} \pm 6.1\times10^{-8}$ |
| Abz-YVADAPK(Dpn)G-OH‡ | $9.6\times10^{-9} \pm 3.2\times10^{-10}$ | |

In conclusion, the present work describes for the first time an alternative assay to characterize inhibitory effects of small molecules on the enzyme activity of meprins. A multiplicity of inhibitors with auto-fluorescence or intercalating properties impedes the screening for effective novel compound. For this purpose, a coupled continuous assay, comprising the auxiliary enzyme dependent conversion of a chromogenic substrate by meprin was established. The hereby introduced assay enables to verify and identify selective inhibitors and discard artifacts of these compounds.

Provided is an improved catalytic assay, using the chromogenic substrate KKGYVADAP-pNA in combination with an auxiliary enzyme to further increase specificity. The serine-protease prolyl tripeptidyl aminopeptidase (PtP) from *Porphyromonas gingivalis* appeared to be a suitable auxiliary enzyme liberating a tripeptide from a free N-terminus (Banbula, A., Mak, P., Bugno, M., Silberring, J., Dubin, A., Nelson, D., Travis, J., Potempa, J., The Journal of biological chemistry. 1999, 274 (14), 9246-9252). The enzyme releases a tripeptide, X-X-P from substrates consisting of a free N-terminus and a proline in the third position (Xu, Y., Nakajima, Y., Ito, K., Zheng, H., Oyama, H., Heiser, U., Hoffmann, T., Gärtner, U.-T., Demuth, H.-U., Yoshimoto, T., Journal of molecular biology. 2008, 375 (3), 708-719, 10.1016/j.jmb.2007.09.077; Ito, K., Nakajima, Y., Xu, Y., Yamada, N., Onohara, Y., Ito, T., Matsubara, F., Kabashima, T., Nakayama, K., Yoshimoto, T., Journal of molecular biology. 2006, 362 (2), 228-240, 10.1016/j.jmb.2006.06.083). The rate-determining step, the cleavage of the first six amino acids Lys-Lys-Gly-Tyr-Val-Ala by meprin $\beta$ and subsequently the release of p-nitroaniline by PtP displays a consistent assay to determine meprin activity. The specificity and accuracy of the coupled assay was evaluated by comparison of inhibitor constants of actinonin, the most potent inhibitor of meprins (Madoux, F., Tredup, C., Spicer, T. P., Scampavia, L., Chase, P. S., Hodder, P. S., Fields, G. B., Becker-Pauly, C., Minond, D., Biopolymers. 2014, 102 (5), 396-406, 10.1002/bip.22527). This assay offers a novel tool to screen for inhibitors independent of fluorogenic substrates, leaving the opportunity to test inhibitors with intercalating or artifactual properties. Developing selective inhibitors are of high interest to validate meprins as important therapeutic targets.

## Claims

1. A method for identifying a compound that inhibits meprin activity, comprising:

   (a) providing a reaction mixture comprising a meprin and an auxiliary enzyme;
   (b) adding a test compound;
   (c) incubating the reaction mixture of step (b);
   (d) adding a labeled substrate to the reaction mixture;
   (e) incubating the reaction mixture of step (d);
   (f) optionally terminating the reaction;
   (g) measuring the amount of free label in the reaction mixture; and
   (h) determining if the test compound inhibits meprin activity by comparing the amount of free label from the reaction mixture with the amount of free label from a control reaction mixture to which no test compound was added, wherein a reduced amount of free label in the reaction mixture compared to the amount of free label in the control reaction mixture is indicative of inhibition of the enzymatic catalytic activity of a meprin by the test compound;

   wherein said labeled substrate is a labeled oligopeptide of formula (I),
   $X_1X_2X_3X_4X_5X_6X_7X_8X_9X_{10}X_{11}$P-label (I)
   wherein
   $X_1$ may be a peptide or any amino acid including proteinogenic amino acids, non-proteinogenic amino acids, L-amino acids and D-amino acids;
   $X_2$, $X_3$, $X_4$, $X_5$, $X_6$, $X_7$, $X_8$ and $X_9$ may be any amino acid including proteinogenic amino acids, non-proteinogenic amino acids, L-amino acids and D-amino acids; and
   at least one of $X_1$, $X_2$, $X_3$, $X_4$, $X_5$, $X_6$, $X_7$, $X_8$ and $X_9$ is present and the others may be absent; and
   the label is selected AMC and pNA;

or
a labeled oligopeptide of formula (II)
$X_4X_5X_6X_7X_8X_9X_{10}X_{11}$P-label (II)
wherein

$X_4$ is any amino acid or a peptide;
$X_5$ is any amino acid;
$X_6$ is any amino acid;
$X_7$ is amino acid selected from the group consisting of A, D, E, I, L, P, S, T, V, and Y;
$X_8$ is amino acid selected from the group consisting of A, D, E, I, L, P, Q, S, T, Q, and V;
$X_9$ is amino acid selected from the group consisting of A, D, E, L, N, Q, S;
$X_{10}$ is amino acid selected from the group consisting of D, E and T;
$X_{11}$ is amino acid selected from the group consisting of A, I, L, V, and F; and

the label is pNA or AMC;
or
a labeled oligopeptide of formula (III)
KKGYVADAP-label (III),
wherein the label is selected from AMC and pNA.

2. The method of claim 1, wherein the IC50 value of a test compound is measured, said comprising the steps (a) to (g) as claimed in claim 1, and wherein step (h) is replaced by step:
(h) determining the IC50 value of the test compound upon the amount of free label in each reaction mixture.

3. The method of claim 1, wherein a competitive inhibitor of a meprin is discriminated from a non-competitive inhibitor of a meprin, said method comprising the steps (a) to (g) as claimed in claim 1, and wherein step (h) is replaced by step:
(h) determining if the meprin inhibitor is a competitive inhibitor of a meprin or a non-competitive inhibitor of a meprin based upon the amount of free label in the reaction mixture.

4. The method according to any one of the preceding claims, wherein said meprin is meprin $\alpha$ or meprin $\beta$, such as human meprin $\alpha$ or human meprin $\beta$.

5. The method of claim 1 or 4, wherein a compound that specifically inhibits meprin $\alpha$ activity is identified, said method comprising the steps (a) to (g) as claimed in claim 1, and wherein step (h) is replaced by step:
(h) determining if the test compound specifically inhibits meprin $\alpha$ activity by comparing the amount of free label from the reaction mixture with the amount of free label from a control reaction mixture which comprises meprin $\beta$, wherein a reduced amount of free label in the reaction mixture compared to the amount of free label in the control reaction mixture is indicative of the specific inhibition of the enzymatic catalytic activity of meprin $\alpha$ by the test compound.

6. The method of claim 1 or 4, wherein a compound that specifically inhibits meprin $\beta$ activity is identified, said method comprising the steps (a) to (g) as claimed in claim 1, and wherein step (h) is replaced by step::
(h) determining if the test compound specifically inhibits meprin $\beta$ activity by comparing the amount of free label from the reaction mixture with the amount of free label from a control reaction mixture which comprises meprin $\alpha$, wherein a reduced amount of free label in the reaction mixture compared to the amount of free label in the control reaction mixture is indicative of the specific inhibition of the enzymatic catalytic activity of meprin $\beta$ by the test compound.

7. A method for assaying enzymatic catalytic activity of a meprin comprising the steps of:

(a) providing a reaction mixture comprising a sample and an auxiliary enzyme;
(b) adding a labeled substrate to the reaction mixture;
(c) incubating the reaction mixture;
(d) optionally terminating the reaction; and
(e) measuring the amount of free label in the reaction mixture, wherein the presence of free label is indicative of enzymatic catalytic activity of a meprin;

wherein said labeled substrate is a labeled oligopeptide of formula (I),

$X_1X_2X_3X_4X_5X_6X_7X_8X_9X_{10}X_{11}$P-label (I)

wherein

$X_1$ may be a peptide or any amino acid including proteinogenic amino acids, non-proteinogenic amino acids, L-amino acids and D-amino acids;

$X_2$, $X_3$, $X_4$, $X_5$, $X_6$, $X_7$, $X_8$ and $X_9$ may be any amino acid including proteinogenic amino acids, non-proteinogenic amino acids, L-amino acids and D-amino acids; and

at least one of $X_1$, $X_2$, $X_3$, $X_4$, $X_5$, $X_6$, $X_7$, $X_8$ and $X_9$ is present and the others may be absent; and

the label is selected AMC and pNA;

or

a labeled oligopeptide of formula (II)

$X_4X_5X_6X_7X_8X_9X_{10}X_{11}$P-label (II)

wherein

$X_4$ is any amino acid or a peptide;

$X_5$ is any amino acid;

$X_6$ is any amino acid;

$X_7$ is amino acid selected from the group consisting of A, D, E, I, L, P, S, T, V, and Y;

$X_8$ is amino acid selected from the group consisting of A, D, E, I, L, P, Q, S, T, Q, and V;

$X_9$ is amino acid selected from the group consisting of A, D, E, L, N, Q, S;

$X_{10}$ is amino acid selected from the group consisting of D, E and T;

$X_{11}$ is amino acid selected from the group consisting of A, I, L, V, and F; and

the label is pNA or AMC;

or

a labeled oligopeptide of formula (III)

KKGYVADAP-label (III),

wherein the label is selected from AMC and pNA.

8. The method according to any one of the preceding claims, wherein said auxiliary enzyme is a prolyl tripeptidyl aminopeptidase (PtP), such as PtP from *Porphyromonas gingivalis.*

9. The method according to any one of the preceding claims, wherein said meprin and said auxiliary enzyme are recombinant enzymes.

10. The method according to any one of the preceding claims, wherein said label is a radioactive, fluorogenic, chromogenic or enzymatic label, such as pNA or AMC.

11. The method according to any one of the preceding claims, wherein said incubation is at a temperature in the range of 25°C to 45°C, preferably at about 30°C and/or wherein said method is performed at a pH value in the range of 6.0 to 8.0, preferably at about 7.4 or 7.5.

12. The method according to any one of the preceding claims, wherein said reaction mixture comprises about 0.2 % dimethyl sulfoxide.

13. The method according to any one of the preceding claims, wherein said method is employed in a high throughput screen.

14. A kit for identifying one or more compounds that modulate meprin activity, comprising meprin $\alpha$ and/or meprin $\beta$, a labeled peptide substrate selected from KKGYVADAP-pNA and/or KKGYVADAP-AMC, the auxiliary enzyme PtP, and instructions for identifying one or more compounds that modulate meprin enzymatic activity.

**Patentansprüche**

1. Verfahren zum Identifizieren einer Verbindung, welche die Meprinaktivität hemmt, das Folgendes umfasst:

(a) das Bereitstellen eines Reaktionsgemisches, das ein Meprin und ein Hilfsenzym umfasst;
(b) das Zugeben einer Testverbindung;
(c) das Inkubieren des Reaktionsgemisches von Schritt (b);
(d) das Zugeben eines markierten Substrats zum Reaktionsgemisch;

(e) das Inkubieren des Reaktionsgemisches von Schritt (d);

(f) gegebenenfalls das Beenden der Reaktion;

(g) das Messen der Menge an freiem Marker im Reaktionsgemisch und

(h) das Bestimmen, ob die Testverbindung die Meprinaktivität hemmt, indem die Menge an freiem Marker aus dem Reaktionsgemisch mit der Menge an freiem Marker aus einem Kontrollreaktionsgemisch verglichen wird, zu dem keine Testverbindung gegeben wurde, wobei eine reduzierte Menge an freiem Marker im Reaktionsgemisch im Vergleich zur Menge an freiem Marker im Kontrollreaktionsgemisch auf eine Hemmung der enzymatischen katalytischen Aktivität eines Meprins durch die Testverbindung hindeutet;

wobei es sich beim markierten Substrat um ein markiertes Oligopeptid der Formel (I),

$X_1X_2X_3X_4X_5X_6X_7X_8X_9X_{10}X_{11}$P-Marker (I),

wobei

$X_1$ ein Peptid oder eine beliebige Aminosäure einschließlich proteinogener Aminosäuren, nichtproteinogener Aminosäuren, L-Aminosäuren und D-Aminosäuren sein kann;

$X_2$, $X_3$, $X_4$, $X_5$, $X_6$, $X_7$, $X_8$ und $X_9$ beliebige Aminosäuren einschließlich proteinogener Aminosäuren, nichtproteinogener Aminosäuren, L-Aminosäuren und D-Aminosäuren sein können und

$X_1$, $X_2$, $X_3$, $X_4$, $X_5$, $X_6$, $X_7$, $X_8$ und/oder $X_9$ vorhanden sind und die anderen nicht vorhanden sein können und der Marker aus AMC und pNA ausgewählt ist;

ein markiertes Oligopeptid der Formel (II)

$X_4X_5X_6X_7X_8X_9X_{10}X_{11}$P-Marker (II),

wobei

$X_4$ eine beliebige Aminosäure oder ein Peptid ist;

$X_5$ eine beliebige Aminosäure ist;

$X_6$ eine beliebige Aminosäure ist;

$X_7$ eine Aminosäure ist, die ausgewählt ist aus der Gruppe bestehend aus A, D, E, I, L, P, S, T, V und Y;

$X_8$ eine Aminosäure ist, die ausgewählt ist aus der Gruppe bestehend aus A, D, E, I, L, P, Q, S, T, Q und V;

$X_9$ eine Aminosäure ist, die ausgewählt ist aus der Gruppe bestehend aus A, D, E, L, N, Q, S;

$X_{10}$ eine Aminosäure ist, die ausgewählt ist aus der Gruppe bestehend aus D, E und T;

$X_{11}$ eine Aminosäure ist, die ausgewählt ist aus der Gruppe bestehend aus A, I, L, V und F; und der Marker pNA oder AMC ist

oder

ein markiertes Oligopeptid der Formel (III)

KKGYVADAP-Marker (III)

handelt, wobei der Marker aus AMC und pNA ausgewählt ist.

2. Verfahren nach Anspruch 1, wobei der IC50-Wert einer Testverbindung gemessen wird, umfassend die Schritte (a) bis (g) nach Anspruch 1 und wobei Schritt (h) durch den folgenden Schritt ersetzt ist:

(h) das Bestimmen des IC50-Werts der Testverbindung auf die Menge an freiem Marker in jedem Reaktionsgemisch.

3. Verfahren nach Anspruch 1, wobei ein kompetitiver Inhibitor eines Meprins von einem nichtkompetitiven Inhibitor eines Meprins unterschieden wird, wobei das Verfahren die Schritte (a) bis (g) nach Anspruch 1 umfasst und wobei Schritt (h) durch den folgenden Schritt ersetzt ist:

(h) basierend auf der Menge an freiem Marker im Reaktionsgemisch das Bestimmen, ob der Meprin-Inhibitor ein kompetitiver Inhibitor eines Meprins oder ein nichtkompetitiver Inhibitor eines Meprins ist.

4. Verfahren nach einem der vorhergehenden Ansprüche, wobei das Meprin Meprin-$\alpha$ oder Meprin-$\beta$ ist, wie humanes Meprin-$\alpha$ oder humanes Meprin-$\beta$.

5. Verfahren nach Anspruch 1 oder 4, wobei eine Verbindung identifiziert wird, welche die Meprin-$\alpha$-Aktivität spezifisch hemmt, wobei das Verfahren die Schritte (a) bis (g) nach Anspruch 1 umfasst und wobei Schritt (h) durch den folgenden Schritt ersetzt ist:

(h) das Bestimmen, ob die Testverbindung die Meprin-$\alpha$-Aktivität spezifisch hemmt, indem die Menge an freiem Marker aus dem Reaktionsgemisch mit der Menge an freiem Marker aus einem Meprin-$\beta$ umfassenden Kontrollreaktionsgemisch verglichen wird, wobei eine reduzierte Menge an freiem Marker im Reaktionsgemisch im Vergleich zur Menge an freiem Marker im Kontrollreaktionsgemisch auf die spezifische Hemmung der enzymatischen katalytischen Aktivität von Meprin-$\alpha$ durch die Testverbindung hindeutet.

6. Verfahren nach Anspruch 1 oder 4, wobei eine Verbindung identifiziert wird, welche die Meprin-$\beta$-Aktivität spezifisch

hemmt, wobei das Verfahren die Schritte (a) bis (g) nach Anspruch 1 umfasst und wobei Schritt (h) durch den folgenden Schritt ersetzt ist:

(h) das Bestimmen, ob die Testverbindung die Meprin-$\beta$-Aktivität spezifisch hemmt, indem die Menge an freiem Marker aus dem Reaktionsgemisch mit der Menge an freiem Marker aus einem Meprin-$\alpha$ umfassenden Kontrollreaktionsgemisch verglichen wird, wobei eine reduzierte Menge an freiem Marker im Reaktionsgemisch im Vergleich zur Menge an freiem Marker im Kontrollreaktionsgemisch auf die spezifische Hemmung der enzymatischen katalytischen Aktivität von Meprin-$\beta$ durch die Testverbindung hindeutet.

7. Verfahren zur Untersuchung der enzymatischen katalytischen Aktivität eines Meprins, das die folgenden Schritte umfasst:

(a) das Bereitstellen eines Reaktionsgemisches, das eine Probe und ein Hilfsenzym umfasst;
(b) das Zugeben eines markierten Substrats zum Reaktionsgemisch;
(c) das Inkubieren des Reaktionsgemisches;
(d) gegebenenfalls das Beenden der Reaktion und
(e) das Messen der Menge an freiem Marker im Reaktionsgemisch, wobei das Vorhandensein von freiem Marker auf eine enzymatische katalytische Aktivität eines Meprins hindeutet;

wobei es sich beim markierten Substrat um ein markiertes Oligopeptid der Formel (I),

$X_1X_2X_3X_4X_5X_6X_7X_8X_9X_{10}X_{11}$P-Marker (I)

wobei

$X_1$ ein Peptid oder eine beliebige Aminosäure einschließlich proteinogener Aminosäuren, nichtproteinogener Aminosäuren, L-Aminosäuren und D-Aminosäuren sein kann;

$X_2$, $X_3$, $X_4$, $X_5$, $X_6$, $X_7$, $X_8$ und $X_9$ beliebige Aminosäuren einschließlich proteinogener Aminosäuren, nichtproteinogener Aminosäuren, L-Aminosäuren und D-Aminosäuren sein können und

$X_1$, $X_2$, $X_3$, $X_4$, $X_5$, $X_6$, $X_7$, $X_8$ und/oder $X_9$ vorhanden sind und die anderen nicht vorhanden sein können und der Marker aus AMC und pNA ausgewählt ist;

oder

ein markiertes Oligopeptid der Formel (II)

$X_4X_5X_6X_7X_8X_9X_{10}X_{11}$P-Marker (II)

wobei

$X_4$ eine beliebige Aminosäure oder ein Peptid ist;

$X_5$ eine beliebige Aminosäure ist;

$X_6$ eine beliebige Aminosäure ist;

$X_7$ eine Aminosäure ist, die ausgewählt ist aus der Gruppe bestehend aus A, D, E, I, L, P, S, T, V und Y;

$X_8$ eine Aminosäure ist, die ausgewählt ist aus der Gruppe bestehend aus A, D, E, I, L, P, Q, S, T, Q und V;

$X_9$ eine Aminosäure ist, die ausgewählt ist aus der Gruppe bestehend aus A, D, E, L, N, Q, S;

$X_{10}$ eine Aminosäure ist, die ausgewählt ist aus der Gruppe bestehend aus D, E und T;

$X_{11}$ eine Aminosäure ist, die ausgewählt ist aus der Gruppe bestehend aus A, I, L, V und F; und

der Marker pNA oder AMC ist;

oder

ein markiertes Oligopeptid der Formel (III)

KKGYVADAP-Marker (III),

handelt, wobei der Marker aus AMC und pNA ausgewählt ist.

8. Verfahren nach einem der vorhergehenden Ansprüche, wobei das Hilfsenzym eine Prolyltripeptidylaminopeptidase (PtP) wie PtP von *Porphyromonas gingivalis* ist.

9. Verfahren nach einem der vorhergehenden Ansprüche, wobei das Meprin und das Hilfsenzym rekombinante Enzyme sind.

10. Verfahren nach einem der vorhergehenden Ansprüche, wobei der Marker ein radioaktiver, flurogener, chromogener oder enzymatischer Marker wie pNA oder AMC ist.

11. Verfahren nach einem der vorhergehenden Ansprüche, wobei die Inkubation bei einer Temperatur im Bereich von 25 °C bis 45 °C, vorzugsweise bei etwa 30 °C, erfolgt und/oder wobei das Verfahren bei einem pH-Wert im Bereich von 6,0 bis 8,0, vorzugsweise bei etwa 7,4 oder 7,5, durchgeführt wird.

**12.** Verfahren nach einem der vorhergehenden Ansprüche, wobei das Reaktionsgemisch etwa 0,2 % Dimethylsulfoxid umfasst.

**13.** Verfahren nach einem der vorhergehenden Ansprüche, wobei das Verfahren in einem Screen mit hohem Durchsatz verwendet wird.

**14.** Kit zum Identifizieren von einer oder mehreren die Meprinaktivität modulierenden Verbindungen, das Meprin-$\alpha$ und/oder Meprin-$\beta$, ein aus KKGYVADAP-pNA und/oder KKGYVADAP-AMC ausgewähltes markiertes Peptidsubstrat, das Hilfsenzym PtP und Anweisungen zum Identifizieren einer oder mehrerer die enzymatische Meprinaktivität modulierenden Verbindungen umfasst.

**Revendications**

**1.** Procédé destiné à identifier un composé qui inhibe l'activité d'une méprine, comprenant les étapes consistant à :

(a) fournir un mélange réactionnel comprenant une méprine et une enzyme auxiliaire ;
(b) ajouter un composé à tester ;
(c) incuber le mélange réactionnel de l'étape (b) ;
(d) ajouter un substrat marqué au mélange réactionnel ;
(e) incuber le mélange réactionnel de l'étape (d) ;
(f) en option, terminer la réaction ;
(g) mesurer la quantité de marqueur libre dans le mélange réactionnel ; et
(h) déterminer si le composé à tester inhibe l'activité d'une méprine en comparant la quantité de marqueur libre provenant du mélange réactionnel avec la quantité de marqueur libre provenant d'un mélange réactionnel témoin auquel aucun composé à tester n'a été ajouté, dans lequel une quantité réduite de marqueur libre dans le mélange réactionnel, en comparaison avec la quantité de marqueur libre dans le mélange réactionnel témoin, est une indication d'une inhibition de l'activité enzymatique catalytique d'une méprine par le composé à tester ;

dans lequel ledit substrat marqué est un oligopeptide marqué de formule (I),
$X_1X_2X_3X_4X_5X_6X_7X_8X_9X_{10}X_{11}$P-marqueur (I) dans laquelle
$X_1$ peut être un peptide ou un quelconque acide aminé, y compris des acides aminés protéinogènes, des acides aminés non protéinogènes, des acides L-aminés et des acides D-aminés ;
$X_2, X_3, X_4, X_5, X_6, X_7, X_8$ et $X_9$ peuvent être un quelconque acide aminé, y compris des acides aminés protéinogènes, des acides aminés non protéinogènes, des acides L-aminés et des acides D-aminés ; et
au moins l'un des $X_1, X_2, X_3, X_4, X_5, X_6, X_7, X_8$ et $X_9$ est présent et les autres peuvent être absents ; et
le marqueur est choisi parmi les AMC et pNA ; un oligopeptide marqué de formule (II)
$X_4X_5X_6X_7X_8X_9X_{10}X_{11}$P-marqueur (II) dans laquelle
$X_4$ est un quelconque acide aminé ou un peptide ;
$X_5$ est un quelconque acide aminé ;
$X_6$ est un quelconque acide aminé ;
$X_7$ est un acide aminé choisi dans le groupe constitué par A, D, E, I, L, P, S, T, V et Y ;
$X_8$ est un acide aminé choisi dans le groupe constitué par A, D, E, I, L, P, Q, S, T, Q et V ;
$X_9$ est un acide aminé choisi dans le groupe constitué par A, D, E, L, N, Q, S ;
$X_{10}$ est un acide aminé choisi dans le groupe constitué par D, E et T ;
$X_{11}$ est un acide aminé choisi dans le groupe constitué par A, I, L, V et F ; et
le marqueur est pNA ou AMC ;
ou
un oligopeptide marqué de formule (III)
KKGYVADAP-marqueur (III),
dans laquelle le marqueur est choisi parmi les AMC et pNA.

**2.** Procédé de la revendication 1, dans lequel la valeur d'IC50 d'un composé à tester est mesurée, comprenant les étapes (a) à (g) tel que revendiqué dans la revendication 1, et dans lequel l'étape (h) est remplacée par une étape :
(h) déterminant la valeur d'IC50 du composé à tester en fonction de la quantité de marqueur libre dans chaque mélange réactionnel.

**3.** Procédé de la revendication 1, dans lequel un inhibiteur compétitif d'une méprine est distingué d'un inhibiteur non compétitif d'une méprine, ledit procédé comprenant les étapes (a) à (g) tel que revendiqué dans la revendication 1, et dans lequel l'étape (h) est remplacée par une étape :

(h) déterminant si l'inhibiteur de méprine est un inhibiteur compétitif d'une méprine ou un inhibiteur non compétitif d'une méprine sur la base de la quantité de marqueur libre dans le mélange réactionnel.

4. Procédé selon l'une quelconque des revendications précédentes, dans lequel ladite méprine est une méprine $\alpha$ ou une méprine $\beta$, telle la méprine $\alpha$ humaine ou la méprine $\beta$ humaine.

5. Procédé des revendications 1 ou 4, dans lequel un composé qui inhibe spécifiquement l'activité d'une méprine $\alpha$ est identifié, ledit procédé comprenant les étapes (a) à (g) tel que revendiqué dans la revendication 1, et dans lequel l'étape (h) est remplacée par une étape :

(h) déterminant si le composé à tester inhibe spécifiquement l'activité d'une méprine $\alpha$, par une comparaison de la quantité de marqueur libre provenant du mélange réactionnel avec la quantité de marqueur libre provenant d'un mélange réactionnel témoin qui comprend une méprine $\beta$, dans lequel une quantité réduite de marqueur libre dans le mélange réactionnel, en comparaison avec la quantité de marqueur libre dans le mélange réactionnel témoin, est une indication de l'inhibition spécifique de l'activité enzymatique catalytique d'une méprine $\alpha$ par le composé à tester.

6. Procédé des revendications 1 ou 4, dans lequel un composé qui inhibe spécifiquement l'activité d'une méprine $\beta$ est identifié, ledit procédé comprenant les étapes (a) à (g) tel que revendiqué dans la revendication 1, et dans lequel l'étape (h) est remplacée par une étape :

(h) déterminant si le composé à tester inhibe spécifiquement l'activité d'une méprine $\beta$, par une comparaison de la quantité de marqueur libre provenant du mélange réactionnel avec la quantité de marqueur libre provenant d'un mélange réactionnel témoin qui comprend une méprine $\alpha$, dans lequel une quantité réduite de marqueur libre dans le mélange réactionnel, en comparaison avec la quantité de marqueur libre dans le mélange réactionnel témoin, est une indication de l'inhibition spécifique de l'activité enzymatique catalytique d'une méprine $\beta$ par le composé à tester.

7. Procédé destiné à doser l'activité enzymatique catalytique d'une méprine comprenant les étapes consistant à :

(a) fournir un mélange réactionnel comprenant un échantillon et une enzyme auxiliaire ;
(b) ajouter un substrat marqué au mélange réactionnel ;
(c) incuber le mélange réactionnel ;
(d) en option, terminer la réaction ; et
(e) mesurer la quantité de marqueur libre dans le mélange réactionnel, dans lequel la présence de marqueur libre est une indication de l'activité enzymatique d'une méprine ;

dans lequel ledit substrat marqué est un oligopeptide marqué de formule (I),
$X_1 X_2 X_3 X_4 X_5 X_6 X_7 X_8 X_9 X_{10} X_{11} P$-marqueur (I) dans laquelle
$X_1$ peut être un peptide ou un quelconque acide aminé, y compris des acides aminés protéinogènes, des acides aminés non protéinogènes, des acides L-aminés et des acides D-aminés ;
$X_2, X_3, X_4, X_5, X_6, X_7, X_8$ et $X_9$ peuvent être un quelconque acide aminé, y compris des acides aminés protéinogènes, des acides aminés non protéinogènes, des acides L-aminés et des acides D-aminés ; et
au moins l'un des $X_1, X_2, X_3, X_4, X_5, X_6, X_7, X_8$ et $X_9$ est présent et les autres peuvent être absents ;
et
le marqueur est choisi parmi les AMC et pNA ;
ou
un oligopeptide marqué de formule (II)
$X_4 X_5 X_6 X_7 X_S X_9 X_{10} X_{11} P$-marqueur (II) dans laquelle
$X_4$ est un quelconque acide aminé ou un peptide ;
$X_5$ est un quelconque acide aminé ;
$X_6$ est un quelconque acide aminé ;
$X_7$ est un acide aminé choisi dans le groupe constitué par A, D, E, I, L, P, S, T, V et Y ;
$X_8$ est un acide aminé choisi dans le groupe constitué par A, D, E, I, L, P, Q, S, T, Q et V ;
$X_9$ est un acide aminé choisi dans le groupe constitué par A, D, E, L, N, Q, S ;
$X_{10}$ est un acide aminé choisi dans le groupe constitué par D, E et T ;
$X_{11}$ est un acide aminé choisi dans le groupe constitué par A, I, L, V et F ; et
le marqueur est pNA ou AMC ;

ou
un oligopeptide marqué de formule (III)
KKGYVADAP-marqueur (III),
dans laquelle le marqueur est choisi parmi les AMC et pNA.

8. Procédé selon l'une quelconque des revendications précédentes, dans lequel ladite enzyme auxiliaire est une prolyl tripeptidyl aminopeptidase (PtP), telle que la PtP provenant de *Porphyromonas gingivalis.*

9. Procédé selon l'une quelconque des revendications précédentes, dans lequel ladite méprine et ladite enzyme auxiliaire sont des enzymes recombinantes.

10. Procédé selon l'une quelconque des revendications précédentes, dans lequel ledit marqueur est un marqueur radioactif, fluorogène, chromogène ou enzymatique, tel que pNA ou AMC.

11. Procédé selon l'une quelconque des revendications précédentes, dans lequel ladite incubation est à une température comprise dans la plage des 25°C à 45°C, de préférence à environ 30°C, et/ou ledit procédé étant exécuté à une valeur de pH comprise dans la plage des 6,0 à 8,0, de préférence à environ 7,4 ou 7,5.

12. Procédé selon l'une quelconque des revendications précédentes, dans lequel ledit mélange réactionnel comprend environ 0,2% de diméthyl sulfoxyde.

13. Procédé selon l'une quelconque des revendications précédentes, ledit procédé étant employé dans un crible à haut débit.

14. Trousse destinée à identifier un ou plusieurs composé(s) qui module/modulent l'activité d'une méprine, comprenant une méprine $\alpha$ et/ou une méprine $\beta$, un substrat peptidique marqué choisi parmi KKGYVADAP-pNA et/ou KKGYVADAP-AMC, l'enzyme auxiliaire PtP et des instructions destinées à identifier un ou plusieurs composé(s) qui module/modulent l'activité enzymatique d'une méprine.

**Figure 1**

**Figure 2**

**Figure 3**

**Figure 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BOND ; BEYNON.** *Protein Sci.,* 1995, vol. 4, 1247-1261
- **ELDERING et al.** *Eur. J. Biochem.,* 1997, vol. 247, 920-932
- **RICARDO et al.** *Am. J. Physiol.,* 1996, vol. 270, F669-676
- **SAMPSON et al.** *J. Biol. Chem.,* 2001, vol. 276, 34128-34188
- **TRACHTMANN et al.** *Biochem. Biophys. Res. Commun.,* 1995, vol. 208, 498-505
- **JIANG et al.** *J. Biol. Chem.,* 1992, vol. 267, 9185-9193
- **BIEN, J. ; JEFFERSON, T. ; CAUSEVIĆ, M. ; JUMPERTZ, T. ; MUNTER, L. ; MULTHAUP, G. ; WEGGEN, S. ; BECKER-PAULY, C. ; PIETRZIK, C. U.** *The Journal of biological chemistry,* 2012, vol. 287 (40), 33304-33313
- **BECKER-PAULY, C. ; PIETRZIK, C. U.** *Frontiers in molecular neuroscience,* 2016, vol. 9, 159
- **BRODER, C. ; BECKER-PAULY, C.** *The Biochemical journal,* 2013, vol. 450 (2), 253-264
- **SCHÜTTE, A. ; ERMUND, A. ; BECKER-PAULY, C. ; JOHANSSON, M. E. V. ; RODRIGUEZ-PINEIRO, A. M. ; BACKHED, F. ; MULLER, S. ; LOTTAZ, D. ; BOND, J. S. ; HANSSON, G. C.** *Proceedings of the National Academy of Sciences of the United States of America,* 2014, vol. 111 (34), 12396-12401
- **PROX, J. ; ARNOLD, P. ; BECKER-PAULY, C.** *Matrix biology journal of the International Society for Matrix Biology,* 2015, vol. 44-46, 7-13
- **BECKER-PAULY, C. ; BARRE, O. ; SCHILLING, O. ; DEM KELLER, U. AUF ; OHLER, A. ; BRODER, C. ; SCHÜTTE, A. ; KAPPELHOFF, R. ; STOCKER, W. ; OVERALL, C. M.** *Molecular & cellular proteomics MCP,* 2011, vol. 10 (9), 9233
- **BERTENSHAW, G. P. ; VILLA, J. P. ; HENGST, J. A. ; BOND, J. S.** *Biological chemistry,* 2002, vol. 383 (7-8), 1175-1183
- **SCHLENZIG, D. ; WERMANN, M. ; RAMSBECK, D. ; MOENKE-WEDLER, T. ; SCHILLING, S.** *Protein expression and purification,* 2015, vol. 116, 75-81
- **MADOUX, F. ; TREDUP, C. ; SPICER, T. P. ; SCAMPAVIA, L. ; CHASE, P. S. ; HODDER, P. S. ; FIELDS, G. B. ; BECKER-PAULY, C. ; MINOND, D.** *Biopolymers,* 2014, vol. 102 (5), 396-406

- **JANSON.** Protein Purification: Principles, High Resolution Methods, and Applications. Wiley, 1997
- **ROSENBERG.** Protein Analysis and Purification: Benchtop Techniques. Birkhauser, 1996
- **WALKER.** The Protein Protocols Handbook. Humana Press, 1996
- **DOONAN.** Protein Purification Protocols. Humana Press, 1996
- **SCOPES.** Protein Purification: Principles and Practice. Springer-Verlag, 1994
- **HARRIS.** Protein Purification Methods: A Practical Approach. IRL Press, 1989
- **SAMBROOK.** Molecular Cloning: A Laboratory Manual. Cold Spring Harbor Laboratory Press, 2001
- **GLOVER.** DNA Cloning: A Practical Approach. IRL Press, 1995
- **GAIT.** Oligonucleotide Synthesis: A Practical Approach. IRL Press, 1984
- **HAMES ; HIGGINS.** Nucleic Acid Hybridisation: A Practical Approach. IRL Press, 1985
- **HAMES ; HIGGINS.** Protein Expression: A Practical Approach. Oxford University Press, 1999
- **MASTERS.** Animal Cell Culture: A Practical Approach. Oxford University Press, 2000
- **BICKERSTAFF.** Immobilization of Cells And Enzymes. Humana Press, 1997
- **PERBAL.** A Practical Guide To Molecular Cloning. Wiley, 1988
- Current Protocols in Molecular Biology. Greene Publishing Associates and Wiley-Interscience, 2002
- **AUSUBEL.** Short Protocols in Molecular Biology: A Compendium of Methods from Current Protocols in Molecular Biology. John Wiley & Sons, 1999
- **ABBENANTE, G. ; LEUNG, D. ; BOND, T. ; FAIRLIE, D. P.** *Lett Pept Sci.,* 2000, vol. 7 (6), 347-351
- **BANBULA, A. ; MAK, P. ; BUGNO, M. ; SILBERRING, J. ; DUBIN, A. ; NELSON, D. ; TRAVIS, J. ; POTEMPA, J.** *The Journal of biological chemistry,* 1999, vol. 274 (14), 9246-9252
- **BRADFORD, M. M.** *Analytical biochemistry,* 1976, vol. 72, 248-254
- **MCCLURE, W. R.** *Biochemistry,* 1969, vol. 8 (7), 2782-2786

- **SCHILLING, S. ; HOFFMANN, T. ; WERMANN, M. ; HEISER, U. ; WASTERNACK, C. ; DEMUTH, H.-U.** *Analytical biochemistry,* 2002, vol. 303 (1), 49-56
- **KRUSE, M.-N. ; BECKER, C. ; LOTTAZ, D. ; KOHLER, D. ; YIALLOUROS, I. ; KRELL, H.-W. ; STERCHI, E. E. ; STOCKER, W.** *The Biochemical journal,* 2004, vol. 378, 383-389
- **XU, Y. ; NAKAJIMA, Y. ; ITO, K. ; ZHENG, H. ; OYAMA, H. ; HEISER, U. ; HOFFMANN, T. ; GÄRTNER, U.-T. ; DEMUTH, H.-U. ; YOSHIMOTO, T.** *Journal of molecular biology,* 2008, vol. 375 (3), 708-719
- **ITO, K. ; NAKAJIMA, Y. ; XU, Y. ; YAMADA, N. ; ONOHARA, Y. ; ITO, T. ; MATSUBARA, F. ; KABASHIMA, T. ; NAKAYAMA, K. ; YOSHIMOTO, T.** *Journal of molecular biology,* 2006, vol. 362 (2), 228-240